# EUROPEAN PATENT APPLICATION

(11) **EP 2 270 233 A1**
(43) Date of publication of application: **05.01.2011**
(21) Application number: 10177454.5
(22) Date of filing: 05.09.2005
(51) Int. Cl.: C12Q 1/68

(54) **Use of both RD9 and IS6110 as nucleic acid targets for the diagnosis of tuberculosis, and provision of multiplex-compliant IS6110 and RD9 targets.**

(62) Divisional of application: 05784765.9
(71) Applicant: Bio-Rad Pasteur, 92430 Marnes-la-Coquette (FR)
(72) Inventor: Clarebout, Gervais, 14550, BLAINVILLE SUR ORNE (FR); Savoye, Chantal, Québec Québec G2G2K1 (CA)
(74) Representative: Paris, Fabienne

(57) **Abstract**

The present invention relates to the use of both RD9 and IS6110 as nucleic acid targets, for the specific and sensitive detection of a mycobacterium of the Mycobacterium tuberculosis complex (MtbC), and/or for the specific and sensitive discrimination of Mycobacterium tuberculosis and Mycobacterium canetti on one hand, from the other strains of said MtbC on the other hand. Advantageously, said MtbC detection and said species discrimination can be made in a single amplification run (multiplex PCR). The means of the invention are advantageous tools for the diagnosis of tuberculosis.

SEQUENCE LISTING

<110>  BIO-RAD PASTEUR

<120>  Use of both RD9 and IS6110 as nucleic acid targets for the
       diagnosis of tuberculosis, and provision of multiplex-compliant
       IS6110 and RD9 targets

<130>  B6377 - FP/KP

<160>  20

<170>  PatentIn version 3.3

<210>  1
<211>  240
<212>  DNA
<213>  Mycobacterium tuberculosis

<400>  1
gctcgaccgg ccagcacgct aattaacggt tcatcgccga tcatcagggc caccgcgagg      60

gccccgatgg tttgcggtgg ggtgtcgagt cgatctgcac acagctgacc gagctgggtg     120

tgccgatcgc cccatcgacc tactacgacc acatcaaccg ggagcccagc cgccgcgagc     180

tgcgcgatgg cgaactcaag gagcacatca gccgcgtcca cgccgccaac tacggtgttt     240

<210>  2
<211>  201
<212>  DNA
<213>  Mycobacterium tuberculosis

<400>  2
cagcacgcta attaacggtt catcgccgat catcagggcc accgcgaggg ccccgatggt      60

ttgcggtggg gtgtcgagtc gatctgcaca cagctgaccg agctgggtgt gccgatcgcc     120

ccatcgacct actacgacca catcaaccgg gagcccagcc gccgcgagct gcgcgatggc     180

gaactcaagg agcacatcag c                                               201

<210>  3
<211>  23
<212>  DNA
<213>  Mycobacterium tuberculosis

<400>  3
cgaccacatc aaccgggagc cca                                              23

<210>  4
<211>  35
<212>  DNA
<213>  Mycobacterium tuberculosis

<400>  4
atgcggcgac cacatcaacc gggagcccac cgcat                                 35

<210>  5
<211>  17
<212>  DNA
<213>  Mycobacterium tuberculosis

<400>  5

cagcacgcta attaccc                                                    17

<210>  6
<211>  17
<212>  DNA
<213>  Mycobacterium tuberculosis

<400>  6
gctgatgtgc tccttga                                                    17

<210>  7
<211>  250
<212>  DNA
<213>  Mycobacterium tuberculosis

<400>  7
tgaccccgca atcggtcatc gccggcacct tcggcggggt aagcgcctgc ggattggccg      60

gtggacgggt cgggttggcc aacgccgtgg ccagcgtgga gtcctcgtaa tagcggacca     120

gtcgccaagc gtagacaccg cggccatagg tggcatcgca ggccgggtat ggccggtagc     180

cggagttcga gccgctttcc agctcaacgc cgctccagtc gaagacggcg gccgaccaac     240

ctggcgcaca                                                           250

<210>  8
<211>  172
<212>  DNA
<213>  Mycobacterium tuberculosis

<400>  8
taagcgcctg cggattggcc ggtggacggg tcgggttggc caacgccgtg gccagcgtgg      60

agtcctcgta atagcggacc agtcgccaag cgtagacacc gcggccatag gtggcatcgc     120

aggccgggta tggccggtag ccggagttcg agccgctttc cagctcaacg cc             172

<210>  9
<211>  24
<212>  DNA
<213>  Mycobacterium tuberculosis

<400>  9
gctattacga ggactccacg ctgg                                            24

<210>  10
<211>  36
<212>  DNA
<213>  Mycobacterium tuberculosis

<400>  10
cgtgcggcta ttacgaggac tccacgctgg cgcacg                               36

<210>  11
<211>  17
<212>  DNA
<213>  Mycobacterium tuberculosis

<400>  11
taagcgcctg cggattg                                                    17

<210> 12
<211> 17
<212> DNA
<213> Mycobacterium tuberculosis

<400> 12
ggcgttgagc tggaaag 17

<210> 13
<211> 92
<212> DNA
<213> Artificial Sequence

<220>
<223> PCR Internal Control

<400> 13
attggatccc agcacgctaa ttacccagta tcagatgacg tggcagccat gagagtggga 60

cagtcgtcct tcaaggagca catcagcgga tc 92

<210> 14
<211> 23
<212> DNA
<213> Artificial Sequence

<220>
<223> PCR probe for Internal Control

<400> 14
gacgtggcag ccatgagagt ggg 23

<210> 15
<211> 20
<212> DNA
<213> Mycobacterium tuberculosis

<400> 15
cgttcaaccc caaacaggta 20

<210> 16
<211> 20
<212> DNA
<213> Mycobacterium tuberculosis

<400> 16
aatcgaactc gtggaacacc 20

<210> 17
<211> 20
<212> DNA
<213> Mycobacterium tuberculosis

<400> 17
ctacctcatc ttccggtcca 20

<210> 18
<211> 20
<212> DNA
<213> Mycobacterium tuberculosis

<400> 18
catagatccc ggacatggtg 20

<210> 19
<211> 1464
<212> DNA
<213> Mycobacterium tuberculosis

<400> 19
tcatggcggc agtaggtaat gcacccaggc gccaccggcg ggcccggcca cggcgtgcag 60
acgggcgttc tgattgcccg ttcggggcag ggtaaagtcc gcgccgatgg ctgtgcaggc 120
tagggcagcc ccggcgaaga ccacgggtgc cggcgtcacg gtccacctgc ctgccgcgtc 180
ccgacaggcc gcagggtgtg ggtcaccgca cgatgcggcg acccagcggc catccgcgcc 240
ctgcagggcg catgctccgg caccggcacg cggttcgtcc ggtgcccagc tccacaacga 300
cgcctgaatg cggccgtctt cggggagcag ctgatcgaag ccgaacagat tgaccccgca 360
atcggtcatc gccggcacct tcggcggggt aagcgcctgc ggattggccg gtggacgggt 420
cgggttggcc aacgccgtgg ccagcgtgga gtcctcgtaa tagcggacca gtcgccaagc 480
gtagacaccg cggccatagg tggcatcgca ggccgggtat ggccggtagc cggagttcga 540
gccgctttcc agctcaacgc cgctccagtc gaagacggcg gccgaccaac ctggcgcaca 600
agacccgacg agcacggctc gtgcgccgga tgcgcggatt tcctcccgcg acacgtcgag 660
tggaagcggg acacagccgt tggtggcacg ccgggccggg ttgggacggt agataaggct 720
tgttccgtcc gcacgccgca acacttggtc gagggtagcc accaccgact catacgccga 780
cgcgttcttc agctggtcct ccaggtagag caggatgacc tcctcggtat gcccgggtgc 840
gttcaaccag ttggcgatct gcggcagcac tgtggccagc agaggttcga cggtgcagcc 900
taggttcgcg ttcttcggtc ccagcccgtg acacacggtg acgccggggg cgccgtggcc 960
ctcgaggcgg ggcaagtagt gcaggtctag ctcgagcgcg cggacgtcga tgtcgagctg 1020
ttgggccaac gacagctgct ggtttgagtc tgcgtgcgag accgtgaacg aatcgctgag 1080
gctgttgaac gagttgtgcg tgccgagcca ctgagtttcc cgcagcggca ccgggtcttg 1140
caacgcatcc tggaaccgcg cggtgcgatg cacccaagac tgtaggtagg catcacgcgc 1200
ggcctgggtc acccggtgcg cgagcggaag cacgcaccgc gcatcgggca caccgacgcg 1260
gcgacactcc gcagcgaccg cgtcggcgaa cttgccgagc gccacgcagg ggatcgcaac 1320
cgggcttatt acgtcacagg atgcggtggg cgagggcgga gcgggcacct ggtaggcatc 1380
ggcggccacc ggtgccgcgg ttatcaacac cacggccaag gcgcccatga gggccgcgct 1440
ctgcagccat cgggcgcggg gcat 1464

<210> 20
<211> 1360
<212> DNA
<213> Mycobacterium tuberculosis

<400> 20
cgatgaaccg ccccggcatg tccggagact ccagttcttg gaaaggatgg ggtcatgtca 60

ggtggttcat cgaggaggta cccgccggag ctgcgtgagc gggcggtgcg gatggtcgca 120
gagatccgcg gtcagcacga ttcggagtgg gcagcgatca gtgaggtcgc ccgtctactt 180
ggtgttggct gcgcggagac ggtgcgtaag tgggtgcgcc aggcgcaggt cgatgccggc 240
gcacggcccg ggaccacgac cgaagaatcc gctgagctga agcgcttagc ggcgggacaa 300
cgccgaattg cgaagggcga acgcgatttt aaagaccgcg tcggctttct tcgcggccga 360
gctcgaccgg ccagcacgct aattaacggt tcatcgccga tcatcagggc caccgcgagg 420
gccccgatgg tttgcggtgg ggtgtcgagt cgatctgcac acagctgacc gagctgggtg 480
tgccgatcgc cccatcgacc tactacgacc acatcaaccg ggagcccagc cgccgcgagc 540
tgcgcgatgg cgaactcaag gagcacatca gccgcgtcca cgccgccaac tacggtgttt 600
acggtgcccg caaagtgtgg ctaaccctga accgtgaggg catcgaggtg gccagatgca 660
ccgtcgaacg gctgatgacc aaactcggcc tgtccgggac cacccgcggc aaagcccgca 720
ggaccacgat cgctgatccg gccacagccc gtcccgccga tctcgtccag cgccgcttcg 780
gaccaccagc acctaaccgg ctgtgggtag cagacctcac ctatgtgtcg acctgggcag 840
ggttcgccta cgtggccttt gtcaccgacg cctacgtcgc aggatcctgg gctggcgggt 900
cgcttccacg atggccacct ccatggtcct cgacgcgatc gagcaagcca tctggacccg 960
ccaacaagaa ggcgtactcg acctgaaaga cgttatccac catacggata ggggatctca 1020
gtacacatcg atccggttca gcgagcggct cgccgaggca ggcatccaac cgtcggtcgg 1080
agcggtcgga agctcctatg acaatgcact agccgagacg atcaacggcc tatacaagac 1140
cgagctgatc aaacccggca agccctggcg gtccatcgag gatgtcgagt tggccaccgc 1200
gcgctgggtc gactggttca accatcgccg cctctaccag tactgcggcg acgtcccgcc 1260
ggtcgaactc gaggctgcct actacgctca acgccagaga ccagccgccg gctgaggtct 1320
cagatcagag agtctccgga ctcaccgggg cggttcacga 1360

## Description

### FIELD OF THE INVENTION:

The present invention relates to tuberculosis, and more particularly to the diagnosis of tuberculosis.
The means of the invention makes use of both RD9 and IS6110 as nucleic acid targets for the diagnosis of tuberculosis.
The present invention notably allows for discriminating between Mycobacterium species. It thus allows an accurate diagnosis, and thereby provides an advantageous tool for the selection of the appropriate treatment.

### SUMMARY OF THE INVENTION:

The present invention relates to tuberculosis, and more particularly to the diagnosis of tuberculosis.
The means of the invention makes use of both RD9 and IS6110 as nucleic acid targets for the diagnosis of tuberculosis.
The present invention thereby provides means which are specially adapted to the detection of mycobacteria that belong to the Mycobacterium tuberculosis complex (MtbC). The means of the invention further enable to discriminate M. tuberculosis and M. canetti on one hand, from the other strains of the MtbC (such as M. bovis) on the other hand.

According to an advantageous feature, the present invention provides multiplex-compliant RD9 and IS6110 targets, which are especially adapted to the implementation of a multiplex PCR, such that said MtbC detection and said species discrimination can be achieved in a single amplification run.
According to the multiplex PCR embodiment, the means of the invention advantageously allow the amplification of two different nucleic acid targets in multiplex, namely a RD9 target and an IS6110 target. With the means of the invention, only one single amplification run is needed to detect a of mycobacterium that belongs to the MtbC, and to discriminate M. tuberculosis and M. canetti on one hand, from the other strains of the MtbC on the other hand. The means of the invention also have the advantage of being of fast and reliable performance: results can be made available on the same day as the day of collection of the patient's sample. They further are specific, sensitive and reliable.

### BACKGROUND OF THE INVENTION:

The World Health Organization estimates that one third of the global population is infected with tuberculosis bacilli, and that 5-10% of people who are infected with tuberculosis bacilli (but who are not infected with HIV) become sick or infectious at some time during their life.
It is estimated that 1.75 million deaths resulted from tuberculosis in 2003.
HIV and tuberculosis bacilli further form a lethal combination, each speeding the other's progress.

Strains that are naturally resistant to an anti-tuberculosis drug have been documented in every country surveyed. For example, M. bovis is naturally resistant to pyrazidamide.
Moreover, strains of tuberculosis bacilli that developed a resistance to major anti-tuberculosis drugs have emerged mainly due to inconsistent, partial or irregular conformance to medical treatment.
A particularly dangerous form of drug-resistant tuberculosis is multidrug-resistant tuberculosis, which is defined as the disease caused by tuberculosis bacilli resistant to at least isoniazid and rifampicin, the two most powerful anti-tuberculosis drugs. Rates of multidrug-resistant tuberculosis are high in some countries, especially in the former Soviet Union, and threaten tuberculosis control efforts.

The bacilli that cause tuberculosis in mammals (human beings and/or animals) are mycobacteria which are grouped within what its known as the Mycobacterium tuberculosis complex (MtbC).
The Mycobacterium tuberculosis complex notably comprises the following mycobacteria: Mycobacterium africanum, Mycobacterium bovis (including the attenuated M. bovis BCG -Bacillus Calmette-Guérin-), Mycobacterium canetti, Mycobacterium caprae, Mycobacterium microti, Mycobacterium pinnipedii, Mycobacterium tuberculosis.

The mycobacteria grouped in the Mycobacterium tuberculosis complex are characterized by 99.9% similarity at the nucleotide level and identical 16S rRNA sequences.

Despite the unusually high degree of conservation in their housekeeping genes, the mycobacteria of the Mycobacterium tuberculosis complex differ widely in terms of host tropisms, phenotypes, and pathogenicity.
Some are exclusively human pathogens (M. tuberculosis, M. africanum, M. canetti), some are exclusively rodent pathogens (M. microti), whereas others can have a wide host spectrum (M. bovis, M. caprae). M. bovis and M. caprae can cause disease in a wide range of domestic or wild animals like cattle or goat, as well as in humans. *M. microti* has been reported to infect small rodents like voles and more recently also human (Niemann et al., 2000 Emerg. Infect. Dis. 6: 539-542; Kubica et al., 2003, J. Clin. Microbiol. 41 3070-3077.). *M. pinnipedii* appears to be the natural host for seal, although the organism is also pathogenic in guinea pigs, rabbits, humans, Brazilian tapir (*Tapirus terrestris*) and, possibly, cattle (Cousin et al., 2003, Int. J. Syst. Evol. Microbiol. 53, 1305-1314).

Fourteen Regions of Difference, ranging in size from 2 to 12.7kb, which are absent from M. bovis BCG, but present in M. tuberculosis H37Rv genome, have been identified. They are referred to as RD1 to RD14 (see Gordon et al. 1999, Mol. Microbiol. 32, 643-655; Behr et al. 1999, Science 284, 1520-1523).
The RD nomenclature which is herein used is that of Brosch et al. (Brosch et al. 2000, Molecular Genetics of Mycobacteria, eds. Hatfull, G.F., & Jacobs, W.R., Jr. (Am. Soc. Microbiol., Washington, DC), pp. 19-36).
Ten of said fourteen BCG⁻ H37Rv⁺ RD regions are highly conserved among M. tuberculosis strains, relative to other members of the Mycobacterium tuberculosis complex (RD1, RD2, RD4, RD7, RD8, RD9, RD10, RD12, RD13, RD14; Brosch et al. 2002, PNAS USA, 99(6), 3684-3689).

Six regions which are absent from the genome of M. tuberculosis H37Rv, relative to other members of the Mycobacterium tuberculosis complex, have been described:
- five H37Rv-related deletions, referred to as RvD1 to RvD5, and
- the region known as M. tuberculosis specific deletion 1 (TbD1), (Gordon et al. 1999, Mol. Microbiol. 32, 643-655; Brosch et al. 1999, Infect. Immun. 67, 5768-5774).

TbD1 was originally identified as a 2,153-bp region that was specifically lacking from the *mmpL6* gene of almost all M. tuberculosis strains (Brosch et al. 2002, PNAS USA 99:3684-3689).
Based on the presence or absence of the TbD1 region, M. tuberculosis strains can be divided into "ancestral" and "modern' types, respectively. The Beijing, Haarlem and African strains responsible for major epidemics are modern types (Kremer et al. 1999, J. Clin. Microbiol. 37, 2607-2618; Brosch et al. 2002, PNAS USA, 99(6), 3684-3689).

Clinical signs and symptoms of tuberculosis are not sufficiently specific to constitute in and of their own a reliable diagnosis of tuberculosis.

Diagnosis is therefore performed through mycobacterial analysis of a biological sample, meant to detect the presence or absence of a mycobacterium belonging to the MtbC.

Various biological and molecular mycobacterial characteristics have been utilized to identify MtbC isolates.

A series of classical tests based upon growth, phenotypic and biochemical properties have been traditionally used to segregate members of the MtbC (Haas et al. 1997, J. Clin. Microbiol. 35, 663-666; Niemann et al. 2000, J. Clin. Microbiol. 38, 3231-3234).

However, these tests can be slow, cumbersome, imprecise, non-reproducible, and time-consuming. They further may not give an unambiguous result in every case, thereby impeding their use as a diagnosis tool for tuberculosis.

Current detection tools for tuberculosis diagnosis notably comprise:
- microscopy (direct observation and/or cell staining),
- cell culture,
- a Gen-Probe® kit.

Mycobacteria are non-motile pleomorphic rods. M. bovis colonies appear smooth and dysgenic (i.e., small colonies), whereas M. tuberculosis colonies are rough and eugenic (i.e., large colonies).
Direct observation of bacteria colonies is however not very specific. Nor is it very sensitive, as the sample shall contain a relatively high concentration in Mycobacterium cells (> 10⁴/mL).

Organisms belonging to the genus Mycobacterium have a unique cell envelope that contains mycolic acids and has high lipid content. Because the cells are hydrophobic and tend to clump together, they are impermeable to the usual stains, such as the Gram stain. They are known as "acid-fast bacilli" because of their lipid-rich cell envelope, which is relatively impermeable to various basic dyes unless the dyes are combined with phenol. Once stained, the cells resist decolorization with acidified organic solvents, and are therefore called "acid-fast". Presently, two types of acid-fast stains are used in clinical mycobacteriology laboratories. One type is carbol fuchsin (Ziehl-Neelsen [ZN] or Kinyoun methods), and the other is fluorochrome (either auramine or auramine-rhodamine).
As a characterising feature, they retain fuchsine or auramine staining after successive or simultaneous treatment with acid and alcohol.
Although the specificity of acid-fast microscopy is excellent for mycobacterial species, the sensitivity is not optimal. The sensitivity of microscopy is influenced by numerous factors, such as the prevalence and severity of disease, the type of specimen, the quality of specimen collection, the number of mycobacteria present in the specimen, the method of processing (direct or concentrated), the method of centrifugation, the staining technique, and the quality of the examination.

It is recommended that a negative result should only be reported following the examination of at least 100 (in low-income countries) and preferably 300 (in industrialized countries) microscopic immersion view fields (or equivalent fluorescent view fields).
Therefore, when microscopy is performed correctly, it can be time-consuming and laborious. False-negativity due to fatigue may also contribute to decreased sensitivity.

Mycobacteria are slow-growing bacilli, with a usual generation time of 12 to 18 hours. Colonies usually become visible only after a 3-week to 10-week incubation time. Samples which contain a low concentration in Mycobacterium cells further necessitate several subcultures. Mycobacteria culture on specific media is a technique that allows the identification of the particular Mycobacterium species contained in the biological sample. It however is time-consuming, especially for those patients who are only at the beginning of the infection process.

Nucleic acid hybridization tests have been developed to detect mycobacteria in a biological sample.
The first tests utilized direct probe hybridization. However, the concentration in Mycobacterium cells contained in a sample collected from a patient is usually too low to give a positive hybridization signal.
Tests utilizing PCR amplification have therefore been developed.
For example, the Gen-Probe® kit commercialized as "Amplified^{™} Mycobacterium tuberculosis direct test" kit, or MTD test kit, (Gen-Probe Inc., San Diego, California 92121, USA) utilizes the amplification of MtbC-specific rRNA (Transcription-Mediated Amplification), followed by amplicon detection in accordance with the Gen-Probe HPA method (Hybridization Protection Assay).
A positive MTD test indicates that the sample contains mycobacteria of the MtbC. Due to problems of inhibition, and to insufficient sensitivity, a negative MTD test does however not exclude the possibility of isolating a MtbC organism from the sample. The MTD test does further not discriminate between the various Mycobacterium species; it more particularly does not discriminate M. bovis from M. tuberculosis.

Diagnosis of tuberculosis is further complicated by the fact that those people who received BCG vaccination are M. bovis positive (e.g., their urine samples are M. bovis positive). Hence, a person can be M. bovis positive, without being a tuberculosis carrier.
Under such circumstances, it is of first importance to detect whether the biological sample contains a MtbC mycobacterium which is not M. bovis, and more particularly which is M. tuberculosis.

Hence, there is a need for a diagnosis tool which would be applicable to samples originating from mammals, and more particularly from human beings, which could discriminate M. tuberculosis from the other main MtbC strains, and more particularly from M. bovis.
There is a need for a quick and reliable MtbC+ diagnosis test, and preferably for a quick and reliable diagnosis of whether the biological sample is susceptible to contain a mycobacterium belonging to the MtbC, which is not M. bovis (MtbC+ Mb-), most preferably a quick and reliable diagnosis of whether the biological sample is susceptible to contain M. tuberculosis cells (Mt+), or whether it does not contain M. tuberculosis cells (Mt-).
The present invention provides such a test and tools. The means of the invention are especially adapted to amplification, especially to PCR amplification, and as an advantageous feature, to multiplex PCR. According to the multiplex PCR embodiment of the present invention, two different nucleic acid targets can be amplified in multiplex, i.e., in a single amplification run: one target is appropriately selected within IS6110, and the other target is appropriately selected within RD9.

IS6110 and RD9 are known to the person of the ordinary skill in the art.
IS6110 sequences have e.g. been described in EP 0 490 951 B1 (US 5,597,911; US 5,807,672; US 5,837,455) and in EP 0 461 045 B1 (US 5,776,693), in the name of INSTITUT PASTEUR. The IS6110 sequence of M. tuberculosis H37Rv (IS6110 reference sequence) is available under accession no. X17348 M29899.
RD9 sequences have been described e.g., in WO 00/55632 in the name of INSTITUT PASTEUR (EP 1 161 562 A1; and US counter-part patent application(s)). The RD9 sequence of M. tuberculosis H37Rv (RD9 reference sequence) comprises the cobL, Rv2073c, Rv 2074 and Rv2075c sequences. The RD9 sequence of M. tuberculosis H37Rv corresponds to the sequence extending from position 250,271 to position 254,176 of the sequence of the genome of M. tuberculosis H37Rv, which is available under accession no. BX842578 (segment 7/13 of M. tuberculosis H37Rv genome).

To the best of the Applicant's knowledge, no prior art however suggests to combine the detection of IS6110 to the detection of RD9; no prior art discloses means that would be appropriate to the detection of IS6110 and RD9 in multiplex PCR; and no prior art discloses the IS6110 and RD9 sub-sequences selected as multiplex-compliant targets in accordance with the present invention (e.g., SEQ ID NO:2 and SEQ ID NO:8 of the present invention).

The means of the invention are especially adapted to the provision of very detailed and accurate mycobacterial information in a single amplification run. The invention thereby provides fast, reliable, specific and sensitive means for the diagnosis of tuberculosis. Diagnosis can thus be fast and accurate, which is of immediate benefit to the patient.
The means of the invention can also be used as tools to assess whether a particular medical treatment may have, or is having any positive effect against the infection. Because they discriminate M. tuberculosis from the other main MtbC strains, and more particularly M. tuberculosis from M. bovis, the means of the invention further enable to take resistance to anti-tuberculosis drugs into due account, and to thereby avoid the administration of inappropriate treatments. They further avoid the consequent development of resistant bacilli.

### SHORT DESCRIPTION OF THE FIGURES:

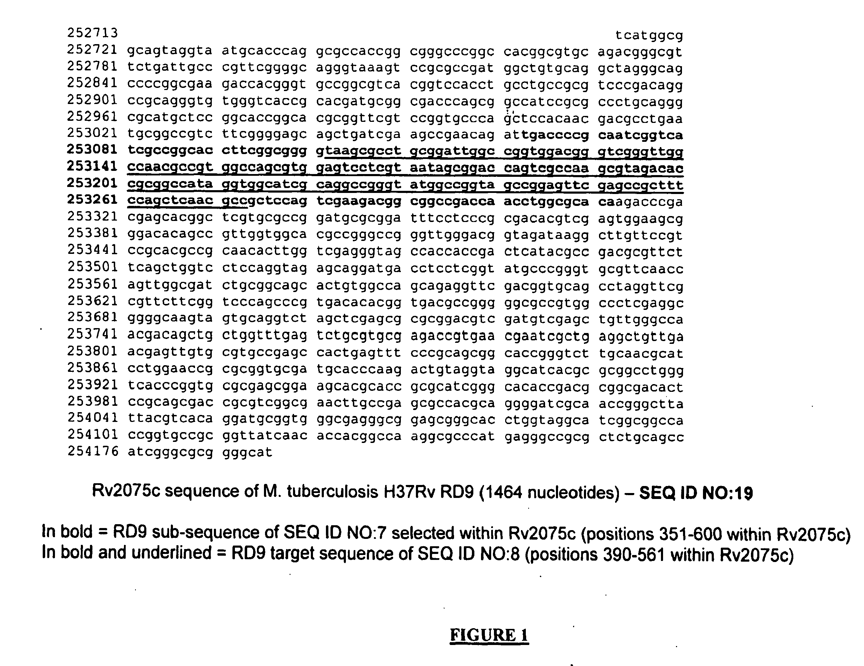

Figure 1 shows a sub-sequence of M. tuberculosis H37RV RD9 (sequence Rv2075c of 1464 nucleotides; SEQ ID NO:19), and, in bold characters, the sequence of SEQ ID NO:7 of the invention (positions 351-600 within Rv2075c).
   The RD9 target sequence of SEQ ID NO:8 of the invention (positions 390-561 within Rv2075c) is shown in bold and underlined characters.

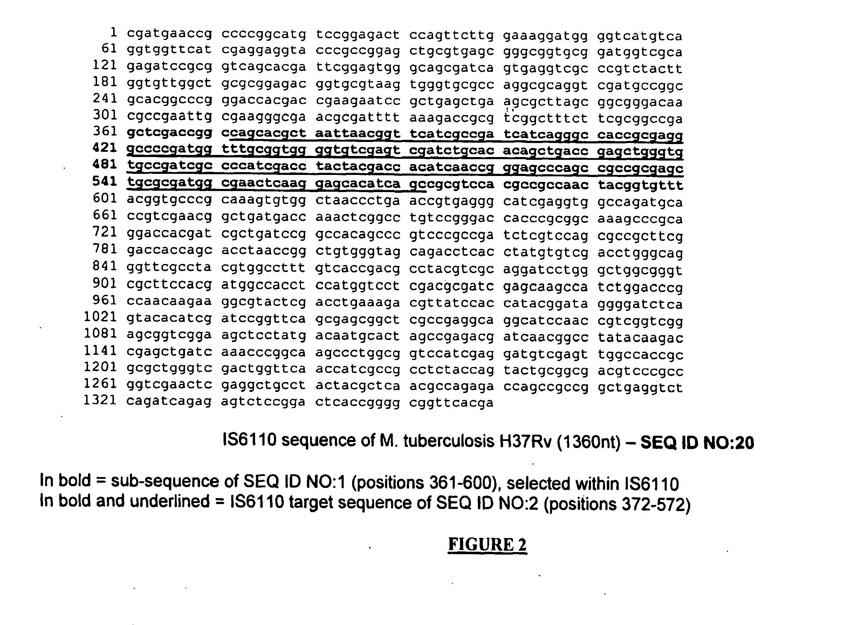

Figure 2 shows the IS6110 sequence of M. tuberculosis H37RV (1360nt; SEQ ID NO:20).
   The sub-sequence of SEQ ID NO:1 (positions 361-600), selected within IS6110 in accordance with the present invention is shown in bold characters.
   The IS6110 target sequence of SEQ ID NO:2 of the invention (positions 372-572) is shown in bold and underlined characters.

### DETAILED DESCRIPTION OF THE INVENTION:

In the present application, each of said Mycobacterium africanum, Mycobacterium bovis, Mycobacterium canetti, Mycobacterium caprae, Mycobacterium microti, Mycobacterium pinnipedii, and Mycobacterium tuberculosis is understood as constituting a distinct species on a nomenclatural point of view. M. bovis comprises M. bovis, as well as the attenuated M. bovis BCG (e.g., M. bovis Pasteur, or M. bovis Tokyo) which derives from M. bovis.
As demonstrated by an extensive literature in the field, each of said species is known to the person of ordinary skill in the art (see e.g. Kremer et al. 1999, J. Clin. Microbiol. 37, 2607-2618; Goh et al. 2001, J. Clin. Microbiol. 39, 3705-3708; Van Soolingen et al. 1998, J. Clin. Microbio. 36, 1840-1845).
Illustrative strains notably comprise:
- ATCC 27294, ATCC 25177, ATCC 51910 for M. tuberculosis,
- ATCC 25420, ATCC 35711 for M. africanum (subtype I),
- ATCC 19422, ATCC 35782, ATCC 11152 for M. microti,
- ATCC 19210, ATCC 35725, ATCC 35726, ATCC 35730 for M. bovis,
- ATCC 27290, ATCC 35736, ATCC 35737 for M. bovis BCG,
- strain CIP 105776 of M. caprae available from the Institut Pasteur Collection (or available from ATCC under number ATCC BAA 824).

ATCC is: American Type Culture Collection, 10801 University Boulevard Manassas, Virginia 20110-2209.
Institut Pasteur Collection or CIP (« Collection de l'Institut Pasteur ») is : Collection de l'Institut Pasteur, Institut Pasteur, 25-28 rue du Docteur Roux, F-75724 Paris Cedex 15, France.

The present invention results from the appropriate selection and use of two Mycobacterium nucleic acids, namely RD9 and IS6110.
IS6110 and RD9 are known to the person of ordinary skill in the art.
IS6110 is an IS-like element, which consists of 1360 nucleotides in M. tuberculosis reference strain H37Rv. The IS6110 sequence of M. tuberculosis H37Rv is available under accession no. X17348 M29899.
RD9 is a Region of Difference, which consists of 1464 nucleotides in M. tuberculosis reference strain H37Rv. The RD9 sequence of M. tuberculosis H37Rv corresponds to positions 250,271-254,176 of the sequence of the genome of M. tuberculosis H37Rv, which is available under accession no. BX842578 (segment 7/13 of M. tuberculosis H37Rv genome).

The use of both RD9 and IS6110 according to the present invention allows the discrimination of M. tuberculosis and M. canetti on one hand, from the other strains of the MtbC, and more particularly from M. bovis, on the other hand.
The use of both RD9 and IS6110allows:
- to detect a Mycobacterium of the MtbC,
- to discriminate M. tuberculosis and M. canetti on one hand, from the other strains of the MtbC on the other hand.

The present invention relates to the use of both RD9 and IS6110 as nucleic acid targets, for the specific and sensitive detection of a mycobacterium of the Mycobacterium tuberculosis complex (MtbC), and/or for the specific and sensitive discrimination of Mycobacterium tuberculosis and Mycobacterium canetti on one hand, from the other strains of said MtbC on the other hand.
The present invention relates to the use of both RD9 and IS6110 as nucleic acid targets, for the *in vitro* diagnosis of tuberculosis.
The present invention thus relates to the use of both:
- at least one IS6110-targeting amplification primer pair or hybridization probe, and
- at least one RD9-targeting amplification primer pair or hybridization probe,
for the specific and sensitive detection of a mycobacterium of the Mycobacterium tuberculosis complex (MtbC), and/or for the specific and sensitive discrimination of Mycobacterium tuberculosis and Mycobacterium canetti on one hand, from the other strains of said MtbC on the other hand. Said use is appropriate for the *in vitro* diagnosis of tuberculosis.
Preferably, said at least one IS6110-targeting amplification primer pair or hybridization probe is a IS6110-specific amplification primer pair or hybridization probe, and said at least one RD9-targeting amplification primer pair or hybridization probe is a RD9-specific amplification primer pair or hybridization probe. By specific primer pair or probe, it is herein meant a primer pair or probe which do not cross-react with any Mycobacterium nucleic acid other than its target (IS6110 or RD9), and preferably which do not cross-react with any human nucleic acid.

The means of the invention are especially adapted to amplification, and more particularly to PCR amplification.
As an advantageous feature, the means of the invention are especially adapted to multiplex PCR. The present invention hence relates to a use of both RD9 and IS6110 as nucleic acid targets, wherein said IS6110 and RD9 are targeted in a multiplex PCR.
According to the multiplex PCR embodiment of the invention, two different nucleic acid targets are targeted for amplification in multiplex, i.e., in a single amplification run: one target is appropriately selected within IS6110 nucleic acid, and the other target is appropriately selected within RD9 nucleic acid.
The specific RD9 and IS6110 nucleic acid targets selected in accordance with the present invention enable:
- to detect a Mycobacterium of the MtbC,
- to discriminate M. tuberculosis and M. canetti on one hand, from the other strains of the MtbC on the other hand, and
- to design primers that can be used in multiplex, i.e., primers that remain specific and sensitive even when used in a single multiplex amplification run.

According to the present invention, when a biological sample is assayed for presence (+) or absence (-) of RD9 and of IS6110, a RD9- IS6110+ response means that the sample contains a mycobacterium of the Mycobacterium tuberculosis complex, which is not M. tuberculosis (i.e., MtbC+ Mt-). Any other RD9 IS6110 response, except RD9- IS6110-, means that the sample contains a mycobacterium of the Mycobacterium tuberculosis complex; which is M. tuberculosis or M. canetti (i.e., MtbC+ and (Mt+ or canetti+)).

**Table 1:**

| Test result (present invention) | Meaning (present invention) |
|---|---|
| RD9- IS6110+ | mycobacterium of the Mycobacterium tuberculosis complex, which is not M. tuberculosis |
| RD9+ IS6110+ | mycobacterium of the Mycobacterium tuberculosis complex, which is M. tuberculosis or M. canetti |
| RD9+ IS6110- | mycobacterium of the Mycobacterium tuberculosis complex, which is M. tuberculosis |
| RD9- IS6110- | not a mycobacterium of the Mycobacterium tuberculosis complex |

The present invention further provides detailed diagnosis within the M. tuberculosis species:
- a RD9+ IS6110- response means that the biological sample contains an ancestral M. tuberculolis strain, and
- a RD9+ IS6110+ response means that the biological sample contains either a modern type M. tuberculosis strain, or the very rare M. canetti.

The invention therefore discriminates ancestral M. tuberculosis strains from modern type M. tuberculosis strains, and avoids the problem of false negative results, which in prior art were sometimes encountered when IS6110 as used as sole marker.

**Table 2:**

| Test result (present invention) | Meaning (present invention) |
|---|---|
| RD9+ IS6110- | ancestral M. tuberculosis |
| RD9+ IS6110+ | modern type M. tuberculosis, or M. canetti |

Ancestral strains are herein defined as M. tuberculosis TbD1+ strains, whereas modern M. tuberculosis strains are defined as M. tuberculosis TbD1- strains.
The sequence of the genes *mmpS6* and *mmpL6* from the ancestral M. tuberculosis strain no. 74 containing the TbD1 region was deposited in the EMBL database under accession no. AJ426486 (the 2,153 bp of TbD1 extend from position 340 to position 2492 of AJ426486).
The PCR method and TbD1 primers described in Brosch et al. 2002, PNAS USA 99:3684-3689 can be used for assessing whether TbD1 is present or absent from a biological sample ; see Table 1 in the supporting material of said Brosch et al. publication, describing TBD1intS.F and TBD1intS.R primers (CGT TCA ACC CCA AAC AGG TA - SEQ ID NO:15 - and AAT CGA ACT CGT GGA ACA CC - SEQ ID NO:16 -, respectively), and TBD1fla1-f and TBD1fla1-R primers (CTA CCT CAT CTT CCG GTC CA - SEQ ID NO:17 - and CAT AGA TCC CGG ACA TGG TG - SEQ ID NO:18 -, respectively).

Examples of modern type M. tuberculosis strains notably comprise M. tuberculosis isolate H37Rv, and M. tuberculosis isolate H37Ra.
The complete genome of M. tuberculosis isolate H37Rv has been sequenced (Cole et al. 1998, Nature, 393, 537-544). The sequence is also available under the ID MTBH37RV, accession number AL123456.
TbD1 (M. tuberculosis specific deletion 1) is absent from about 87% of the M. tuberculosis strains, including representative strains from major epidemics such as the Haarlem, Beijing and Africa clusters (Kremer et al. 1999, J. Clin. Microbiol. 37, 2607-2618; Brosch et al. 2002, PNAS USA, 99(6), 3684-3689).

M. canetti has a rare prevalence among human patients showing tuberculosis-like symptoms. Hence, a RD9+ IS6110+ response will most frequently mean MtbC+ and Mt+ (i.e., a mycobacterium of the Mycobacterium tuberculosis complex, which is M. tuberculosis).

If it were nevertheless wished to discriminate between presence of M. tuberculosis (i.e., Mt+) and presence of M. canetti (i.e., canetti+), appropriate means are available to the person of ordinary skill in the art.

Indeed, M. canetti is a very rare, smooth variant of M. tuberculosis, and is usually isolated from patients from, or with connection to, Africa.
Although it shares identical 16S rRNA sequences with the other members of the M. tuberculosis complex, and contain all RD and RvD regions, as well as the TbD1 region, M. canetti strains differ in many respects, including polymorphisms in certain house-keeping genes, IS1081 copy number, colony morphology, and the lipid content of the cell wall, and notably:
i. M. canetti has been shown to carry 26 unique spacer sequences in the DR region that are no longer present in any other member of the M. tuberculosis complex (Van Embden et al. 2000, J. Bacteriol. 182, 2393-2401);
ii. M. canetti can be characterized by the absence of a specific RD^{can} region, described in Brosch et al. 2002, PNAS USA, 99(6), 3684-3689, which partially overlaps RD12, and is sometimes also referred to as RD12-can deletion. The RD12-can deletion sequence of M. canetti isolate 14000059, relative to M. tuberculosis H37Rv genome, was deposited in the EMBL database under accession no. AJ583833;
iii. M. canetti can be characterized by the presence of a single copy of the IS1081 insertion sequence (M. bovis IS1081 is available under accession no. X61270);
iv. M. canetti cells form smooth colonies, whereas M. tuberculosis cells form rough colonies.

Hence, in any situation where the person of ordinary skill in the art would like to discriminate M. tuberculosis from M. canetti, any one of, or several of features i. to iv. can be used.

To summarize:

**Table 3:**

| **Mycobacterium tuberculosis complex** | | | | |
|---|---|---|---|---|
| | | TbD1 | **RD9** | **IS6110** |
| | Ancestral | | | |
| | (East African - Indian family) | + | + | - |
| | Modern | | | |
| M. tuberculosis | (Latin American and Mediterranean family) | - | + | + |
| M. africanum | | + | - | + |
| M. bovis | | + | - | + |
| M. bovis - BCG | | + | - | + |
| M. canetti | | + | + | + |
| M. caprae | | + | - | + |
| M. microti | | + | - | + |
| M. pinnipedii | | + | - | + |

The patterns shown in the above table 3 are typical ones, i.e., those observed in the large majority of, if not all, the strains which belong to the indicated species.

The present invention further provides selected nucleic acid targets within said RD9 and IS6110 nucleic acids.
These selected RD9 and IS6110 nucleic acid targets have a very high level of specificity, as required for diagnosis applications: their detection reliably indicates that RD9 and IS6110 nucleic acids are present in the assayed sample, without confusion with any other nucleic acid, should it be of microbial or mammal origin. These selected targets advantageously allow the design of primers which can be used in multiplex (i.e., in the same amplification run), while still remaining very sensitive and very specific.

Such primers do not lead to cross-hybridization, thereby retaining the required specificity, and remain as sensitive as one Mycobacterium CFU per sample (see example below).

The selected RD9 and IS6110 nucleic acid targets of the present invention have the sequence:
- of SEQ ID NO:2 for the IS6110 target, or the sequence which is fully complementary thereto over the entire length of said SEQ ID NO:2,
- of SEQ ID NO:8 for the RD9 target, or the sequence which is fully complementary thereto over the entire length of said SEQ ID NO:8.
The IS6110 target of SEQ ID NO:2 is a fragment of M. tuberculosis H37Rv IS6110 (accession no. X17348 M29899), from position 372 to 572 (start and end positions included): 
372 cagcacgct aattaacggt tcatcgccga tcatcagggc caccgcgagg
421 gccccgatgg tttgcggtgg ggtgtcgagt cgatctgcac acagctgacc gagctgggtg
481 tgccgatcgc cccatcgacc tactacgacc acatcaaccg ggagcccagc cgccgcgagc
541 tgcgcgatgg cgaactcaag gagcacatca gc
(= SEQ ID NO:2 = from 372 to 572)
 The RD9 target of SEQ ID NO:8 is a fragment of M. tuberculosis H37Rv RD9. The RD9 sequence of M. tuberculosis H37Rv genome extends from position 250,271 to position 254,176 of the H37Rv sequence available under accession no. BX842578. The Rv2075c sequence, which is contained within the RD9 sequence of M. tuberculosis H37Rv, extends from position 252,713 to position 254,176 of the H37Rv sequence available under accession no. BX842578. The RD9 target of SEQ ID NO:8 extends from position 390 to 561 (start and end positions included) of said Rv2075c sequence, namely:

390 t aagcgcctgc
401 ggattggccg gtggacgggt cgggttggcc aacgccgtgg ccagcgtgga
451 gtcctcgtaa tagcggacca gtcgccaagc gtagacaccg cggccatagg
501 tggcatcgca ggccgggtat ggccggtagc cggagttcga gccgctttcc
551 agctcaacgc c
(= SEQ ID NO:8 = from 390 to 561)

The present invention thus relates to a set of two polynucleotides suitable for use as reference template sequences in the design of primers that can be used in multiplex in a single amplification run to detect a mycobacterium of the Mycobacterium tuberculosis complex (MtbC), and/or to discriminate Mycobacterium tuberculosis and Mycobacterium canetti on one hand, from the other strains of said MtbC on the other hand,
wherein one of said two reference template polynucleotides is a fragment of RD9, the other being a fragment of IS6110,
wherein said IS6110 fragment consists in the sequence of SEQ ID NO:2, or in the sequence which is fully complementary to SEQ ID NO:2 over the entire length of SEQ ID NO:2,
and
wherein said RD9 fragment consists in the sequence of SEQ ID NO:8, or the sequence which is fully complementary to SEQ ID NO:8 over the entire length of SEQ ID NO:8.

Said IS6110 fragment advantageously is the fragment extending from position 372 to 572 of the IS6110 sequence of M. tuberculosis strain H37Rv, start and end positions included (M. tuberculosis H37Rv IS6110 sequence is available under accession no. X17348 M29899).
Said RD9 fragment advantageously is the fragment extending from position 390 to 561 of the RD9 sequence of M. tuberculosis strain H37Rv, start and end positions included (the sequence of segment 7/13 of M. tuberculosis H37Rv genome is available under accession no. BX842578; RD9 corresponds to positions 252,713-254,176 of this sequence).

Primers designed from such reference template sequences so that they can lead to the amplification of said selected IS6110 target of SEQ ID NO:2, and to the amplification of said selected RD9 target of SEQ ID NO:8, can be used in multiplex in a single amplification run while still offering a specific and sensitive detection and discrimination.

The present invention also relates to each of said reference template sequences individually, i.e., to a polynucleotide suitable for use as a reference template sequence in the design of primers that can be used in multiplex in a single amplification run to detect a mycobacterium of the Mycobacterium tuberculosis complex (MtbC), and/or to discriminate Mycobacterium tuberculosis and Mycobacterium canetti on one hand, from the other strains of said MtbC on the other hand,
wherein said reference template polynucleotide is selected from said set of two polynucleotides.

The present invention thereby provides specific and sensitive amplification primers and probes which remain sensitive and specific when used in multiplex, and which thereby enable to achieve said MtbC detection and said species discrimination in a single amplification run.
These primers and probes advantageously are designed by reference to said IS6110 and RD9 targets.

Amplification methods, especially polymerase chain reaction (PCR) and PCR-based methods are known in the art (Molecular Cloning: A Laboratory Manual, Maniatis, Fritsch, and Sambrook, CSHL Press; Molecular Biology of the Cell, Alberts et al.; PCR Primer: A Laboratory Manual, Dieffenbach and Dveksler, CSHL Press; The Polymerase Chain Reaction, Mullis, Ferré, and Gibbs, Birkhauser Boston Press; Gene quantification, Ferré, Birkhauser Boston Press.)

The present invention describes nucleic acids (oligonucleotides and polynucleotides) which are especially adapted to the implementation of a multiplex PCR on a biological sample, and more particularly of an at least duplex PCR (two targets, i.e., IS6110 and RD9), most preferably of an at least triplex PCR (two targets, i.e., IS6110 and RD9, and one Internal Control).

By multiplex PCR, we hereby understand any PCR reaction aiming at the amplification of more than one target. For instance, multiplex PCR include duplex PCR (two targets), triplex PCR (three targets), and higher multiplex PCR.

Naturally, the nucleic acids according to the present invention are also suitable for other protocols, including simplex protocols, multiplex protocols, end-point protocols, qualitative protocols, quantitative protocols, combinations thereof, and the like.

By nucleic acid, we hereby understand any nucleic acid: it can be synthetic or not, recombinant or naturally occurring, linear or circular. This includes DNA and RNA. The nucleic acid can be either single stranded or double stranded or even triple stranded. It can stem from various biological sources, such as micro organisms (bacteria and the like), or higher organisms, like mammal cells (e.g. human cells, or non-human mammal cells). The nucleic acid can also comprise total DNA, total RNA, genomic DNA, mitochondrial DNA, plasmidic DNA, BAC DNA, and mixtures thereof. Moreover, the nucleic acid can assume various states of purity.
By sample, we hereby understand any kind of sample, naturally occurring or not. Preferably, said sample is from biological origin. Said sample may also stem from a cell culture supernatant. In a preferred embodiment, said sample is or derives from a bronchial aspirate, a bronchial lavage, sputum, lung tissues, cerebro-spinal fluid, blood, urine.
Said sample might also result from a preliminary step. For instances said sample might be obtainable via a purification and/or extraction procedure. In particular, said sample may result from a separation and/or purification and/or extraction process carried out on a biological sample.
Said sample can also be a control sample. Control samples include samples as qualitative control, positive control, negative control, quantitative control, and calibrating control. Said control can be internal as well as external. Any sample according to the present invention can be present several times. For instance, said sample can be provided as duplicate, as triplicate, as quadruplicate, as multiplicate, which is advantageous in the case of quantitative experiments.

The skilled person is familiar with the notion of target template. Said target template can be any nucleic acid, whose presence is to be assessed in said method. Said target template is possibly, but not necessarily, the nucleic acid to be amplified in said method (PCR amplicon).

By polynucleotide, we hereby understand any polymer of nucleotides, wherein nucleotides can be ribonucleotides, deoxyribonucleotides, dideoxyribonucleotides, degenerated nucleotides, and the like. Said nucleotides are preferably single-stranded, but can also be double stranded. The length of said polynucleotides can vary, and is usually under 500 nucleotides (nt), preferably in the range of 50-400 nt, more preferably 100-300 nt, even more preferably 150-250 nt. For example, the selected IS6110 target of SEQ ID NO:2 is of 201 nt, and the selected RD9 target of SEQ ID NO:8 is of 172nt.

By oligonucleotide, we hereby understand any short polymer of nucleotides, wherein nucleotides can be ribonucleotides, deoxyribonucleotides, dideoxyribonucleotides, degenerated nucleotides, and the like. Said oligonucleotides are preferably single-stranded. The length of said oligonucleotides can vary, and is usually under 150 nucleotides (nt), preferably in the range of 10-100 nt, more preferably 15-60 nt, even more preferably 18-50 nt. Said oligonucleotides can bear chemical modifications, such as tagging or marking, for instance radioactive, fluorescent, biotinylated, dig labelling.
An oligonucleotide according to the invention can be either forward (sense) or reverse (antisense). In addition, it should be stressed, that although preferred functions may be mentioned in relation to some oligonucleotides according to the present invention, it is obvious that a given oligonucleotide may assume several functions, and may be used in different ways according to the present invention. For example, an oligonucleotide can be used either as a primer, or as a probe. Also, when an oligonucleotide is described as being useful as a probe, the skilled person understands that the complementary sequence of this oligonucleotide is equally useful as a probe to target the same amplicon. Moreover, it is also obvious, that any primer suitable for a multiplex assay, can also, within the meaning of the present invention, be used in a simplex protocol.
Oligonucleotides according to the invention especially include PCR primers and probes. Unless otherwise stated, nucleic acid sequences are given in the 5' to 3' direction. Said oligonucleotides can be under many forms e.g., under dry state, in solution/suspension with the desired solvent and the desired concentration. The skilled person would know, which solvents, concentrations, storage conditions are suitable for the oligonucleotides of the invention. In particular, the skilled person would know how to prepare said oligonucleotides as stock solutions. The oligonucleotides according to the invention can also assume various degrees of purity, as can be judged by those skilled in the art, e.g. by HPLC chromatography. In the present application, when a product, such as a nucleic acid, a polynucleotide, an oligonucleotide, a primer, a probe, an amplicon, a reference template sequence, etc., is said to comprise a defined sequence, the scope of such an expression is to be understood as also encompassing the situation where said product consists in said defined sequence.

The notion of primer or PCR primer is known to those skilled in the art. For example, it includes an oligonucleotide able to anneal to a target template under suitable stringency conditions, and allowing for polymerase strand elongation. The typical length of a primer is 10-30 nt, preferably 10-18nt, most preferably 15-18nt, e.g., 15, 16, 17 or 18nt.
Suitable stringency conditions notably comprise conditions of high stringency. Illustrative conditions of high stringency are temperature and ionic conditions used during nucleic acid hybridization and/or washing. "Highly stringent conditions" as well as factors affecting the rate of hybridization are known to those of ordinary skill in the art, and can be found in, for example, Maniatis et al. 1982, Molecular Cloning, Cold Spring Harbor Laboratory. Generally, highly stringent conditions are selected to be about 5 to 20°C lower than the thermal melting point (Tm) of the oligonucleotide at a defined ionic strength and pH. The Tm is the temperature (under defined ionic strength and pH) at which 50% of a complementary target sequence hybridizes to a perfectly matched probe. For DNA - - DNA hybrids, the Tm can be approximated from the equation of Meinkoth and Wahl (Anal. Biochem. 1984, 138:267-284): Tm = 69.3 + 0.41x(CG)%-650/L, wherein L is the strength of the probe in nucleotides. "Highly stringent conditions" also refer to a washing procedure.
Other illustrative conditions of stringency are given in the examples below.

The notion of probe is also known to those skilled in the art. For example, it includes an oligonucleotide able to anneal to a target template under the desired hybridization conditions. The typical length of a probe is 15-60 nt, preferably 15-40 nt, more preferably 15-30 nt, most preferably 15-28 nt, e.g. 16-27 nt. Preferably, said probe is fluorescently labelled. However, it is clear to those skilled in the art that under certain conditions, one may use a primer as a probe and vice-versa. Moreover, it is herein stressed that the products according to the present invention, especially, inter alia, oligonucleotides, are not limited to the intended use herein mentioned, but rather are to be broadly construed, irrespective of the indicated destination. For instance, a claim to a product (oligonucleotide) for a particular use should be construed as meaning a product (oligonucleotide) which is in fact suitable for the stated use. Thus, an oligonucleotide suitable for use as a primer in a multiplex protocol is also clearly adapted to a simplex protocol within the meaning of the present invention.
Various formats (types) of probes, including Taqman™ probes (hydrolysis probes), molecular beacons (beacon probes or molecular beacon probes), and Scorpion™ probes are known in the art.
Nucleic acid probes may comprise intercalating agents, such as one or several intercalating dye(s), within their nucleotide sequence.

In a preferred embodiment, the probes according to the invention can all be synthesized and used in the molecular beacon format.
The structure of molecular beacons is as follows. A short nucleotide sequence (so-called beacon arm) which is unrelated to the target sequence is thus covalently linked to both ends of the probe. A short unrelated arm is thus linked in 5' of the probe, and is labelled with a fluorescent moiety (i.e. fluorescent dye or fluorescent marker). Another but still unrelated arm is linked to the 3' end of probe and is labelled with a fluorescence quenching moiety. Thus, molecular beacons have a fluorophore and a quencher at opposite ends. The 5' short arm is totally complementary to the one in 3' so that they can anneal together, and thus can assume a hairpin structure when unhybridized to the target in solution. In this hairpin conformation, the quencher and the fluorescent dye are close enough to each other to allow efficient quenching of the fluorophore. However, when the probe encounters a target molecule, annealing is favoured with respect to the hairpin conformation when values of beacon arm Tm and probe Tm are suitably chosen (theoretically: probe Tm > beacon arm Tm > primer Tm, wherein Tm is the melting temperature of interest).The fluorophore and quencher move away from each other and the fluorophore can then fluoresce when illuminated by suitable light excitation. As PCR proceeds, amplification product accumulates, and the amount of fluorescence at any given cycle depends on the amount of amplification product present at that time. (See e.g., Sanjay Tyagi and Fred Russell Kramer, Nature Biotechnology 1996, volume 14, pages 303-308; Nature Biotechnology 1998, volume 16, pages 49-53).
It is of course also possible to link the fluorophore at the 3' end, while attaching the quencher at the 5' end.
Schematically, said probe can have the following formulae (molecular beacon format):
5' Fluorophore-(arm1)-probe-(arm2)-Quencher 3'
5' Quencher-(arm1)-probe-(arm2)-Fluorophore 3'
wherein arm1 and arm2 can be any short nucleotide sequences, e.g., in the range of 3-10 nucleotides, preferably 5, 6, 7 nucleotides, allowing for the hair pin structure formation under suitable stringency conditions, i.e., arm1 and arm2 are totally complementary to anneal under the desired stringency conditions (standard PCR stringency conditions include, for example, an annealing temperature of 55 to 65°C and an Mg concentration of 4 to 8 mM). However, arm1 and arm2 are unrelated to the target sequence of the probe, i.e., the hairpin conformation resulting from the annealing between arm1 and arm2 is essentially the only possible secondary structure for the probe when unhybridized. The skilled person would know how to choose such arms for a given probe.
For example, possible beacon formats include:
CGTGCG-(probe sequence)-CGCACG
ATGCGG-(probe sequence)-CCGCAT

By fluorophore, it is herein understood any fluorescent marker/dye known in the art. Examples of such suitable fluorescent markers include Fam, Hex, Tet, Joe, Rox, Tamra, Max, Edans, Cy dyes such as Cy5, Fluorescein, Coumarin, Eosine, Rhodamine, Bodipy, Alexa, Cascade Blue, Yakima Yellow, Lucifer Yellow and Texas Red (all of them are Trade-Marks).
By quencher, we herein understand any quencher known in the art. Examples of such quenchers include Dabcyl, Dark Quencher, Eclipse Dark Quencher, ElleQuencher, Tamra, BHQ and QSY (all of them are Trade-Marks).
The skilled person would know which combinations of dye/quencher are suitable when designing a probe.
In a preferred embodiment according to the invention, spectral properties of said probes can be chosen as to not interfere with each other. In particular, when probes are used in multiplex, each single probe can have its own fluorophore being spectrally significantly different from each other i.e., the absorption/emission spectra are essentially non-overlapping. This advantageously allows for low-noise multiplex detection for all single probes, making sure that individual signals do not interfere with each other in detection. Examples of dyes which can be used together in multiplex include Fam with Tamra, Fam with Tamra with Texas Red. According to the invention, all the provided oligonucleotides and polynucleotides can be either kept separately, or partially mixed, or totally mixed.
Said oligonucleotides and polynucleotides can be provided under dry form, or solubilized in a suitable solvent, as judged by the skilled person. Suitable solvents include TE, PCR-grade water, and the like.

The present invention relates to a primer pair which has been designed so that it can lead to the amplification of said selected IS6110 target of SEQ ID NO:2, and to a primer pair which has been designed so that it can lead to the amplification of said selected RD9 target of SEQ ID NO:8.

The present invention thus relates to a set of primers, which comprises at least two pairs of primers. Said at least two pairs of primers are suitable for the specific and sensitive detection of a mycobacterium of the Mycobacterium tuberculosis complex (MtbC), and/or for the specific and sensitive discrimination of Mycobacterium tuberculosis and Mycobacterium canetti on one hand, from the other strains of said MtbC on the other hand. Said at least two pairs of primers have such sequences that they can be used in multiplex in a single amplification run while still offering a specific and sensitive detection and discrimination.
Said at least two pairs of primers are an IS6110 primer pair consisting of an IS6110 forward primer and of an IS6110 reverse primer, and a RD9 primer pair consisting of a RD9 forward primer and of a RD9 reverse primer.
Said RD9 forward primer is a 5'-terminal fragment of 10 to 18 nucleotides of the sequence of SEQ ID NO:8, said 5'-terminal fragment starting from the very first 5' nucleotide of said SEQ ID NO:8.
Said RD9 reverse primer is a 5'-terminal fragment of 10 to 18 nucleotides of the sequence which is fully complementary of said SEQ ID NO:8 over the entire length of said SEQ ID NO:8, said 5'-terminal fragment starting from the very first 5' nucleotide of said complementary sequence.
Said IS6110 forward primer is a 5'-terminal fragment of 10 to 18 nucleotides of the sequence of SEQ ID NO:2, said 5'-terminal fragment starting from the very first 5' nucleotide of said SEQ ID NO:2.
Said IS6110 reverse primer is a 5'-terminal fragment of 10 to 18 nucleotides of the sequence which is fully complementary of said SEQ ID NO:2 over the entire length of said SEQ ID NO:2, said 5'-terminal fragment starting from the very first 5' nucleotide of said complementary sequence.
Each of said primers preferably is a fragment of 10 to 18 nucleotides, 11 to 18 nucleotides, 12 to 18 nucleotides, 13 to 18 nucleotides, 14 to 18 nucleotides, 15 to 18 nucleotides, 16 to 18 nucleotides, 17 to 18 nucleotides, e.g., 17 nucleotides. Said RD9 forward primer may advantageously comprise the sequence of SEQ ID NO:11 (RD389 primer).
Said RD9 reverse primer may advantageously comprise the sequence of SEQ ID NO:12 (RD561 primer).
Said IS6110 forward primer may advantageously comprise the sequence of SEQ ID NO:5 (IS368 primer).
Said IS6110 reverse primer may advantageously comprise the sequence of SEQ ID NO:6 (IS569 primer).
Preferably, said IS6110 forward and reverse primers are (independently of each other) of 10-18 nucleotides e.g., of 10, 11, 12, 13, 14, 15, 16, 17, or 18 nucleotides. Most preferably, said IS6110 forward and reverse primers are (independently of each other) of 14-18 nucleotides e.g., of 15-18, 15-17, for example 15, 16 or 17 nucleotides.
Preferably, said RD9 forward and reverse primers are (independently of each other) of 10-18 nucleotides e.g., of 10, 11, 12, 13, 14, 15, 16, 17, or 18 nucleotides. Most preferably, said RD9 forward and reverse primers are (independently of each other) of 14-18 nucleotides e.g., of 15-18, 15-17, for example 15, 16 or 17 nucleotides.
The present invention also relates to each of said primer pairs, and each of said primer, as an individually entity.
The present invention thus relates to a primer pair selected from said set of at least two primer pairs (i.e., a RD9 primer pair, or an IS6110 primer), and to a primer selected from such a primer pair (i.e., a RD9 forward primer, a RD9 reverse primer, an IS6110 forward primer, an IS6110 reverse primer).

Using a primer pair which has been designed so that it can lead to the amplification of said selected IS6110 target of SEQ ID NO:2, and a primer pair which has been designed so that it can lead to the amplification of said selected RD9 target of SEQ ID NO:8, as amplification primers on a biological sample containing a MtbC Mycobacterium which is different from M. tuberculosis reference strain H37Rv, will lead to the production of an IS6110 amplicon and/or of an RD9 amplicon, the respective sequences of which may slightly vary from the reference sequences of SEQ ID NO:2 and/or SEQ ID NO:8, respectively, depending on the nucleic acid sequence of the particular MtbC Mycobacterium strain present in said sample.
For example:
- when the present invention is implemented on a biological sample which contains a MtbC Mycobacterium which is a modern type M. tuberculosis, both a RD9 and a IS6110 amplicon will be obtained,
- when the present invention is implemented on a biological sample which contains a MtbC Mycobacterium which is an ancestral M. tuberculosis, a RD9 amplicon, but no IS6110 amplicon, will be obtained,
- when the present invention is implemented on a biological sample which contains a MtbC Mycobacterium which is not M. tuberculosis, and which is not M. canetti, an IS6110 amplicon, but no RD9 amplicon, will be obtained.

As soon as the MtbC Mycobacterium contained in said biological sample is not M. tuberculosis H37Rv, the obtained amplicons may have variant sequences, relative to said IS6110 and RD9 reference sequences of SEQ ID NO:2 and NO:8.
Such variant sequences will have approximately the same size as the reference sequences of SEQ ID NO:2 or SEQ ID NO:8, respectively.
For example, an IS6110 amplicon obtainable by implementation of the present invention will have a size ranging from 190 to 210 nucleotides, preferably from 195 to 205 nucleotides, most preferably from 199 to 203 nucleotides, and an RD9 amplicon obtainable by implementation of the present invention will have a size ranging from 160 to 185 nucleotides, preferably from 165 to 180 nucleotides, most preferably from 170 to 174 nucleotides.
Such variant sequences will show a high degree of identity with said reference sequences of SEQ ID NO:2 or SEQ ID NO:8, respectively.
For example, an IS6110 amplicon obtainable by implementation of the present invention will share a minimum of 95% sequence identity with the reference sequence of SEQ ID NO:2, and a RD9 amplicon obtainable by implementation of the present invention will share a minimum of 95% sequence identity with the reference sequence of SEQ ID NO:8. Preferably, said minimum sequence identity will be of 96%, 97%, 98%, or 99%. Said identity % are calculated over the entire length of said reference sequence of SEQ ID NO:2 or SEQ ID NO:8, respectively. Such variant sequences having a high degree of identity may alternatively be characterized by the fact that they hybridize to said IS6110 reference sequence of SEQ ID NO:2, or to said RD9 reference sequence of SEQ ID NO:8, under highly stringent conditions. "Highly stringent conditions" as well as factors affecting the rate of hybridization are known to those of ordinary skill in the art, and can be found in, for example, Maniatis et al. 1982, Molecular Cloning, Cold Spring Harbor Laboratory. Illustrative "highly stringent conditions" for example comprise hybridization in 6xSSC or 6xSSPE at 68°C in aqueous solution or at 42°C in the presence of 50% formamide, and comprise washing conditions of said hybridized variant sequence with said reference sequence in an aqueous solution containing 0.1xSSC and 0.2 SDS, at room temperature for 2-60 min, followed by incubation in a solution containing 0.1xSSC at a temperature about 12-20°C below the calculated Tm of the hybrid detected, for 2-60 min. Other illustrative highly stringent conditions can be found in the examples below.

Hence, the present invention also relates to such IS6110 and/or RD9 amplicons obtainable by implementation of the present invention on a biological sample containing a MtbC Mycobacterium, and more particularly a MtbC Mycobacterium which is different from M. tuberculosis reference strain H37Rv. The present invention more particularly relates to:
- any polynucleotide which has a size ranging from 190 to 210 nucleotides, preferably from 195 to 205 nucleotides, most preferably from 199 to 203 nucleotides, and which
   o shares a minimum of 95% sequence identity with the reference sequence of SEQ ID NO:2 or with the sequence which is fully complementary to SEQ ID NO:2 over the entire length of SEQ ID NO:2 (preferably, a minimum sequence identity of 96%, 97%, 98%, or 99%), and/or
   o hybridize to the reference sequence of SEQ ID NO:2 or with the sequence which is fully complementary to SEQ ID NO:2 over the entire length of SEQ ID NO:2 under highly stringent conditions, and to
- any polynucleotide which has a size ranging from 160 to 185 nucleotides, preferably from 165 to 180 nucleotides, most preferably from 170 to 174 nucleotides, and which
   o shares a minimum of 95% sequence identity with the reference sequence of SEQ ID NO:8 or with the sequence which is fully complementary to SEQ ID NO:8 over the entire length of SEQ ID NO:8 (preferably, a minimum sequence identity of 96%, 97%, 98%, or 99%), and/or
   o hybridize to the reference sequence of SEQ ID NO:8 or with the sequence which is fully complementary to SEQ ID NO:8 over the entire length of SEQ ID NO:8 under highly stringent conditions.

The present invention relates to probes which are suitable for the detection of the amplicons obtainable by using an IS6110 primer pair and a RD9 primer pair as primers in an amplification reaction performed on a biological sample. Advantageously, said probe is:
- the sequence of SEQ ID NO:8, or the sequence which is fully complementary to SEQ ID NO:8 over the entire length of SEQ ID NO:8, or a fragment of at least 15 nucleotides of said sequence of SEQ ID NO:8 or of said complementary sequence, or
- the sequence of SEQ ID NO:2, or the sequence which is fully complementary to SEQ ID NO:2 over the entire length of SEQ ID NO:2, or a fragment of at least 15 nucleotides of said sequence of SEQ ID NO:2 or of said complementary sequence.

Preferably, said fragment of at least 15 nucleotides of said SEQ ID NO:2 or SEQ ID NO: 8 is of less than 30 nucleotides, most preferably it is of 15-28 nucleotides e.g., of 15, 16, 17, 18, 19, 20, 21, 22, 23, 24, 25, 26, 27 or 28 nucleotides, most preferably of 20-25 nucleotides e.g., of 23 nucleotides.
The present invention more particularly relates to the following amplicon-targeted probes:
- the sequence of SEQ ID NO:8, or the sequence which is fully complementary to SEQ ID NO:8 over the entire length of SEQ ID NO:8, or a fragment of at least 15 nucleotides and of less than 29 nucleotides of said sequence of SEQ ID NO:8 or of said complementary sequence, or
- the sequence of SEQ ID NO:2, or the sequence which is fully complementary to SEQ ID NO:2 over the entire length of SEQ ID NO:2, or a fragment of at least 15 nucleotides and of less than 29 nucleotides of said sequence of SEQ ID NO:2 or of said complementary sequence.
An advantageous probe suitable for the detection of an RD9 amplicon is a fragment of the sequence that is complementary to SEQ ID NO:8, wherein said fragment comprises the sequence of SEQ ID NO:9 (RD441 probe). Preferably, this RD9-targeted probe has less than 30 nucleotides, most preferably 15-28 nucleotides e.g., of 15, 16, 17, 18, 19, 20, 21, 22, 23, 24, 25, 26, 27 or 28 nucleotides, most preferably of 20-25 nucleotides, e.g. of 23 nucleotides.
An advantageous probe suitable for the detection of an IS6110 amplicon, **characterized in that** it is a fragment of SEQ ID NO:2 which comprises the sequence of SEQ ID NO:3 (IS503 probe). Preferably, this IS6110-targeted probe has less than 30 nucleotides, most preferably 15-28 nucleotides e.g., of 15, 16, 17, 18, 19, 20, 21, 22, 23, 24, 25, 26, 27 or 28 nucleotides, most preferably of 20-25 nucleotides e.g., of 23 nucleotides.
The probes of the invention may comprise one or several intercalating agent(s) within their sequence, such as one or several intercalating dye(s).
The probes of the invention may comprise at least one beacon arm, preferably two beacon arms, one at each of its terminal ends (e.g., CGTGCG-(probe sequence)-CGCACG; or ATGCGG-(probe sequence)-CCGCAT). Advantageous beaconed probes notably comprises the probe of SEQ ID NO:10 (which is the probe of SEQ ID NO:9 in beacon format), and the probe of SEQ ID NO:4 (which is the probe of SEQ ID NO:3 in beacon format).
A probe of the invention may comprise a fluorophore at its 5'end and/or a quencher at its 3' end, for example Tamra or Fam at its 5' end, and/or Dabcyl at its 3' end.

The present invention also relates to a PCR set, consisting of at least one primer pair of the invention and of at least one probe of the invention. It more particularly relates to a PCR set consisting of at least one RD9 primer pair of the invention and of at least one RD9 probe of the invention, and to a PCR set consisting of at least one IS6110 primer pair of the invention and of at least one IS6110 probe of the invention.

Suitable internal control (IC) which are especially adapted to the implementation of the invention are also provided.
The present invention relates to oligonucleotides and polynucleotides suitable for use as an Internal Control (IC) in the specific and sensitive detection of a mycobacterium of the Mycobacterium tuberculosis complex (MtbC), and/or for the specific and sensitive discrimination of Mycobacterium tuberculosis and Mycobacterium canetti on one hand, from the other strains of said MtbC on the other hand.
An IC oligonucleotide or polynucleotide of the present invention advantageously comprises:
a. two primer binding site sequences, wherein the one of said two primer binding site sequences which is located in 5' relative to the other of said two primer binding site sequences is identical to one member of a primer pair of the invention (a IS6110 primer pair or a RD9 primer pair), and the other of said two primer binding site sequences is fully complementary to the other member of the same primer pair of the invention, and
b. a sequence which is unrelated to Mycobacterium, which is located between said two primer binding site sequences.
Preferably, said Mycobacterium-unrelated sequence is also not related to any human nucleic acid, most preferably not related to mammal any nucleic acid.

An illustrative IC may consist in 92 bases:
- 17 bases located in the 5' end region, and 17 bases located in the 3' end region, which are identical, and respectively, fully complementary to the IS6110 primers, respectively (IS368 and IS569 of SEQ ID NO:5 and 6, respectively),
- the 58 remaining bases being unrelated to mycobacteria (random sequence).
For example, a kit of the invention may comprise an IC oligonucleotide or polynucleotide which is amplified by an IS6110 primer pair, such as the IC oligonucleotide of SEQ ID NO:13.

The present invention further provides IC probes which are especially adapted to the detection of an IC oligonucleotide or polynucleotide of the invention (by annealing to said control sequence). An illustrative IC probe is the probe of SEQ ID NO:14 (SIM ATTO 590).

The present invention also relates to a kit for the specific and sensitive detection of a mycobacterium of the Mycobacterium tuberculosis complex (MtbC), and/or for the specific and sensitive discrimination of Mycobacterium tuberculosis and Mycobacterium canetti on one hand, from the other strains of said MtbC on the other hand, and to a kit for the *in vitro* diagnosis of tuberculosis.
A kit of the present invention comprises:
- at least one set of two pairs of primers of the invention, and/or
- at least one primer pair of the invention, and/or
- at least one primer of the invention, and/or
- at least one set of two reference template oligonucleotides of the invention, and/or
- at least one reference template polynucleotide of the invention, and/or
- at least one probe of any one of the invention.
A kit of the invention may notably comprise a PCR set of the invention (i.e., at least one primer pair of the invention, and at least one probe of the invention).
In the kit according to the invention, the nucleic acids (primers, probes) can be either kept separately, or partially mixed, or totally mixed.

Said nucleic acids can be provided under dry form, or solubilized in a suitable solvent, as judged by the skilled person. Suitable solvents include TE, PCR-grade water, and the like.
Such reagents are known to those skilled in the art, and include water, like nuclease-free water, DNAse-free water, PCR-grade water; salts, like magnesium, potassium; buffers such as Tris; enzymes, including polymerases, such as Taq. Vent, Pfu (all of them Trade-Marks), activable polymerase, reverse transcriptase, and the like; nucleotides like deoxynucleotides, dideoxunucleotides, dNTPs, dATP, dTTP, dCTP, dGTP, dUTP; other reagents, like DTT and/or RNase inhibitors; and polynucleotides like polyT, polydT, and other oligonucleotides, e.g. primers.
The kit according to the invention may comprise PCR controls. Such controls are known in the art, and include qualitative controls, positive controls, negative controls, internal controls (IC), quantitative controls, internal quantitative controls, as well as calibration ranges. The internal control for said PCR step can be a template which is unrelated to the target template in the PCR step. Such controls also may comprise control primers and/or control probes. For example, in the case of MtbC Mycobacterium detection, it is possible to use as internal control, a polynucleotide chosen within a gene whose presence is excluded in a sample originating from a human body (for example, from a plant gene), and whose size and GC content is equivalent to those from the target sequence.
A kit of the invention may contain an IC oligonucleotide or polynucleotide of the invention, and/or an IC probe of the invention.
A kit according to the invention may contain means for extracting and/or purifying nucleic acid from a biological sample, e.g. from bronchial aspirate, bronchial lavage, sputum, cerebro-spinal fluid, urine, blood, serum, plasma. Such means are well known to those skilled in the art.
A kit of the invention may comprise a lysis buffer for nucleic acid extraction, for example a lysis buffer containing an anionic resin which binds divalent ions.
Lysis is preferably performed in the presence of detergents and in the presence of an anionic resin which binds divalent ions such as the Chelex X-100 beads. As an advantageous feature, said anionic resin also also is a chaototropic agent.
Examples of appropriate lysis buffer comprises the lysis buffer containing 8% of Chelex X-100 resin (Bio-Rad, ref :142-1253), 0.5 % NP-40 (Sigma, ref : Igepal I-3021) , 0.5 % Tween 20 (VWR, ref : 28829296) in Tris 10 mM buffer (Sigma, ref : T-6791), EDTA 1 mM, (Sigma, ref: E-1644) pH 8.3.
The kit according to the invention may contain instructions for the use thereof. Said instructions can advantageously be a leaflet, a card, or the like. Said instructions can also be present under two forms: a detailed one, gathering exhaustive information about the kit and the use thereof, possibly also including literature data; and a quick-guide form or a memo, e.g. in the shape of a card, gathering the essential information needed for the use thereof.
In a preferred embodiment, said kit is a diagnostics kit, especially an in vitro diagnostics kit, i.e. an tuberculosis diagnostics kit.

The present invention also relates to a method for detecting a mycobacterium of the Mycobacterium tuberculosis complex (MtbC) in a biological sample, and/or for discriminating Mycobacterium tuberculosis and Mycobacterium canetti on one hand, from the other strains of said MtbC on the other hand.
A method of the invention comprises the detection of two target nucleic acid sequences, wherein one of said two target sequences is IS6110 or a fragment thereof, and the other target sequence is RD9 or a fragment thereof,
wherein the detection of the presence of said RD9 target and of the presence or absence of said IS6110 target is indicative of the presence in said biological sample of a mycobacterium belonging to the MtbC, which is either M. tuberculosis or M. canetti,
wherein the detection of the absence of said RD9 target and of the presence of said IS6110 target is indicative of the presence in said biological sample of a mycobacterium belonging to the MtbC, which is not M. tuberculosis,
wherein the detection of the absence of said RD9 target and the absence of said IS6110 target is indicative of the absence in said biological sample of a mycobacterium belonging to the MtbC.

The detection of the presence of said RD9 target, and of the presence or absence of said IS6110 target is notably indicative of the presence in said biological sample of a mycobacterium belonging to the MtbC, which is not M. bovis.

Advantageously, notably for a multiplex PCR implementation of the method, said RD9 target sequence consists in the sequence of SEQ ID NO:8, or in the sequence which is fully complementary to SEQ ID NO:8 over the entire length of SEQ ID NO:8, and said IS6110 target sequence consists in the sequence of SEQ ID NO:2, or in the sequence which is fully complementary to SEQ ID NO:2 over the entire length of SEQ ID NO:2.

The detection of the presence of said RD9 target and of the absence of said IS6110 target is indicative of the presence in said biological sample of a mycobacterium belonging to the MtbC, which is either M. canetti, or an ancestral M. tuberculosis strain.
The detection of the presence of said RD9 target and of the presence of said IS6110 target is indicative of the presence in said biological sample of a mycobacterium belonging to the MtbC, which is either M. canetti, or a modern type M. tuberculosis strain.
The detection of the absence of said RD9 target and of the presence of said IS6110 target is indicative of the presence in said biological sample of a mycobacterium belonging to the MtbC, which is M. bovis, M. africanum, M. caprae, M. microti, or M. pinnipedii.
According to an advantageous feature of the invention, the method can be implemented by PCR.
According to a very advantageous feature of the invention, the method can be implemented in multiplex PCR, notably by using at least one primer pair of the invention (at least one IS6110 and/or at least one RD9 primer pair) as amplification primers.
A method of the invention may comprise the use of at least one probe of the invention as a hybridization probe and/or as a detection probe.

The present invention relates to an in vitro method for the diagnosis of tuberculosis, **characterized in that**
the presence or absence of a mycobacterium of the MtbC in a biological sample, and/or
the presence or absence of a M. tuberculosis or M. canetti strain in a biological sample, and/or
the presence or absence of a mycobacterium of the MtbC, which is not M. tuberculosis, in a biological sample,
is(are) determined by implementation of said method of MtbC detection and/or of MtbC species discrimination on said biological sample.

The invention further provides a method to extract nucleic acid from a biological sample for MtbC analysis.
Lysis is preferably performed in the presence of detergents and in the presence of an anionic resin which binds divalent ions such as the Chelex X-100 beads. As an advantageous feature, said anionic resin also also is a chaototropic agent. Examples of appropriate lysis buffer comprises the lysis buffer containing 8% of Chelex X-100 resin (Bio-Rad, ref :142-1253), 0.5 % NP-40 (Sigma, ref: Igepal I-3021) , 0.5 % Tween 20 (VWR, ref : 28829296) in Tris 10 mM buffer (Sigma, ref: T-6791), EDTA 1 mM, (Sigma, ref: E-1644) pH 8.3.

The present invention is illustrated by the examples below, given on illustrative purposes only.

### EXAMPLE 1 (in vitro bacterial samples - specificity and sensitivity):

### 1. Material and Methods:

### 1.1. Extraction of nucleic acid:

Bacterium cells were collected from 100 different strain cultures (54 bacterial strains other than Mycobacterium; 26 Mycobacterium strains which do not belong to the MtbC; 5 strains of M. bovis, including 4 M. bovis BCG; 23 strains of M. tuberculosis; 6 strains other than M. tuberculosis or M. bovis, but belonging to the MtbC *-M. pinnipedii, M. caprae, 3 strains of M. africanum, M. microti*-; 1 strain of M. canetti).

Lysis is performed in the presence of detergents and in the presence of Chelex X-100 beads (i.e., an anionic resin which binds divalent ions). As an advantageous feature, said anionic resin also is a chaototropic agent.

Composition of lysis buffer: 8% of Chelex X-100 resin (Bio-Rad, ref :142-1253), 0.5 % NP-40 (Sigma, ref : Igepal I-3021), 0.5 % Tween 20 (VWR, ref : 28829296) in Tris 10 mM buffer (Sigma, ref: T-6791), EDTA 1 mM, (Sigma, ref: E-1644) pH 8.3.

### Procedure:

- 200 µl of biological sample
- 400 µl of lysis buffer
- add 10 µl of liquid internal control (IC)
- vortex 30"
- incubation 10 min at 95°C
- centrifugation 5 min 8000 rpm
- PCR on 10 µl of supernatant

The negative control is achieved by replacing the 200 µl of biological sample, by 200 µl of phosphate buffer, pH 6.8 at 25 mM.

The IC is added at the extraction stage. It is under liquid form. Ten µl of IC are added to the 200 µl of biological sample. The IC solution contains 9.6 10⁴ copies of IC in 10 µl, so as to obtain 1.6 10³ copies per PCR test after extraction (1/60 dilution).

### 1.2. Primers and probes:

### 1.2.1. IS6110 system

EMBL data on IS6110 =
LOCUS MTIS6110 1360 bp DNA linear BCT 07-JUL-2002
DEFINITION Mycobacterium tuberculosis IS6110 IS-like element.
ACCESSION X17348 M29899
VERSION X17348.1 GI:48695
KEYWORDS insertion element IS6110; transposon.
SOURCE Mycobacterium tuberculosis H37Rv

Sub-sequence selected within IS6110 =

361 gctcgaccgg ccagcacgct aattaacggt tcatcgccga tcatcagggc caccgcgagg
421 gccccgatgg tttgcggtgg ggtgtcgagt cgatctgcac acagctgacc gagctgggtg
481 tgccgatcgc cccatcgacc tactacgacc acatcaaccg ggagcccagc cgccgcgagc
541 tgcgcgatgg cgaactcaag gagcacatca gccgcgtcca cgccgccaac tacggtgttt
(= SEQ ID NO:1 = from 361 to 600)

Underlined in the above SEQ ID NO:1, are shown the sequences of the primers and probe which are used in the IS6110 system:
- from 372 to 387: IS368 forward primer;
- from 506 to 528: IS503 probe;
- from 556 to 572: complementary sequence of IS569 reverse primer.

IS6110 amplification target sequence in single stranded format =

372 cagcacgct aattaacggt tcatcgccga tcatcagggc caccgcgagg
421 gccccgatgg tttgcggtgg ggtgtcgagt cgatctgcac acagctgacc gagctgggtg
481 tgccgatcgc cccatcgacc tactacgacc acatcaaccg ggagcccagc cgccgcgagc
541 tgcgcgatgg cgaactcaag gagcacatca gc
(= SEQ ID NO:2 = from 372 to 572)

### IS6110 probe:

IS503 probe = CGACCACATCAACCGGGAGCCCA **(SEQ ID NO:3)**
Molecular Beacon® arms = ATGCGG and CCGCAT
Reporter dye and quencher = Tamra® and Dabcyl®
IS503 in Beacon® format: IS503MB probe =
5'-Tamra-ATGCGGCGACCACATCAACCGGGAGCCCACCGCAT- Dabcyl-3' (SEQ ID NO:4)

### IS6110 forward primer:

IS368 primer = 5'-CAGCACGCTAATTACCC-3' **(SEQ ID NO:5)**
IS6110 reverse primer:
   IS569 primer = 5'-GCTGATGTGCTCCTTGA-3' **(SEQ ID NO:6)**

### 1.2.2. RD9 system:

LOCUS BX842578 1464 bp DNA linear BCT 10-JUN-2004
DEFINITION Mycobacterium tuberculosis H37Rv complete genome; segment 7/13.
ACCESSION BX842578 REGION: complement (252713..254176)

From position 250,271 to position 254,176: sequence of RD9

From position 252,713 to position 254,176 of BX842578: sequence of Rv2075c (sub-sequence of RD9).

Sub-sequence selected within RD9 (sub-sequence selected within the Rv2075c sequence contained within RD9):

351 tgaccccgca atcggtcatc gccggcacct tcggcggggt aagcgcctgc
401 ggattggccg gtggacgggt cgggttggcc aacgccgtgg ccagcgtgga
451 gtcctcgtaa tagcggacca gtcgccaagc gtagacaccg cggccatagg
501 tggcatcgca ggccgggtat ggccggtagc cggagttcga gccgctttcc
551 agctcaacgc cgctccagtc gaagacggcg gccgaccaac ctggcgcaca
(= SEQ ID NO:7 = from 351 to 600)

Underlined in the above SEQ ID NO:7, are shown the sequences of the primers and probe which are used in the RD9 system:
- from 390 to 406: RD389 forward primer;
- from 441 to 464: complementary sequence of RD441 probe;
- from 545 to 561: complementary sequence of RD561 reverse primer.

RD9 amplification target sequence in single stranded format =

390 t aagcgcctgc
401 ggattggccg gtggacgggt cgggttggcc aacgccgtgg ccagcgtgga
451 gtcctcgtaa tagcggacca gtcgccaagc gtagacaccg cggccatagg
501 tggcatcgca ggccgggtat ggccggtagc cggagttcga gccgctttcc
551 agctcaacgc c
(= SEQ ID NO:8 = from 390 to 561)

### RD9 probe:

RD441 probe = GCTATTACGAGGACTCCACGCTGG **(SEQ ID NO:9)**
Molecular Beacon® arms = CGTGCG and CGCACG
Reporter dye and quencher = Fam and Dabcyl®
RD441 under Beacon® format: RD441 MB probe =
5'-Fam-CGTGCGGCTATTACGAGGACTCCACGCTGGCGCACG-Dabcyl-3' **(SEQ ID NO:10)**

### RD9 forward primer:

RD389 = 5' -TAAGCGCCTGCGGATTG-3' **(SEQ ID NO:11)**

### RD9 reverse primer:

RD 561 = 5' -GGCGTTGAGCTGGAAAG- 3' **(SEQ ID NO:12)**

### 1.3. Internal Control (IC):

The Internal Control (IC) consists in a single stranded sequence. The IC is selected so as to comprise a sequence which is unrelated to the amplification target sequences, said unrelated sequence being flanked in 5' and 3' by sequences which are appropriate target sequences for one of the primer pairs that is used in the PCR reaction (IS6110 primer pair, or RD9 primer pair).

The IC comprises a sequence in its 5' end region, and another sequence in its 3' end region, wherein said 3'-contained sequence hybridizes to one member of the IS6110 primer pair, and said 5'-contained sequence hybridizes to the complementary sequence of the other member of the same IS6110 primer pair. Preferably, said 3'-contained sequence is fully complementary to one member of the IS6110 primer pair, and said 5'-contained sequence has the same sequence as the other member of the same IS6110 primer pair.
Alternatively, a RD9 primer pair can be used instead of an IS6110 primer pair: the purpose is to have an IC that is amplified by one of the primer pairs which are implemented in the PCR run, but which comprises between said 5'-contained and 3'-contained sequence a sequence which is unrelated to Mycobacterium, preferably unrelated to bacterial and mammal nucleic acids.

The IC of the present example contains in its 5'-end region a sequence which is identical to one member of the IS6110 primer pair, and its 3'-end region, a sequence which fully complementary to the other member of the same IS6110 primer pair.

The IC of the present example consists in 92 bases:
- 17 bases located in the 5' end region and 17 bases located in the 3' end region, which are identical and, respectively, fully complementary to the IS6110 primers (IS368 and IS569 of SEQ ID NO:5 and 6, respectively),
- the 58 remaining bases being unrelated to mycobacteria (random sequence).
The IC of the present example has the following sequence:

ATTGGATCCCAGCACGCTAATTACCCAGTATCAGATGACGTGGCAGCCATGA
GAGTGGGACAGTCGTCCTTCAAGGAGCACATCAGCGGATC (SEQ ID NO:13)

Underlined within the above SEQ ID NO:13, are shown (from 5' to 3'):
- IS368 (IS6110 forward primer of SEQ ID NO:5),
- the IC probe, and
- the target sequence of IS569 (i.e., the complementary sequence of the IS6110 reverse primer of SEQ ID NO:6).

IC probe = SIM ATTO 590 = GACGTGGCAGCCATGAGAGTGGG (SEQ ID NO:14), to which beacon arms are added at its 5' and 3' terminal ends.

### 1.4. Illustrative experimental conditions for triplex PCR:

### Reagents:

Hot Start Taq Polymérase Qiagen (5U/µl, ref 203205), containing the PCR buffer
dNTP :Promega ref U151x (4X 25 mM)
MgCl₂: Sigma, ref. M-2670
PVP10 : Sigma, ref: PVP-10
Glycerol : VWR, ref : 24388295

### Thermocycler.

iQ1 (Bio-Rad)

### Composition of the 1X PCR mix:

In the 1X PCR mix, add: 0.2 µM of RD9 probe in beacon format (SEQ ID NO:10), 0.2 µM of IC probe (SEQ ID NO:14) in beacon format, 0.4 µM of IS6110 probe in beacon format (SEQ ID NO:4), 0.5 µM of each of said four primers, 5% glycerol, 0.3 % PVP 10, 2U of Taq Polymerase, 1 mM dNTP and 5 mM final of MgCl₂.
Ten microlitres of DNA are added to this mix.

### Thermocycling:

- First cycle: 15' at 95°C,
- Repeated 50 times:

| | |
|---|---|
| Second cycle: | 30" at 95°C |
| Third cycle: | 30" at 59°C |
| Fourth cycle: | 30" at 72°C |

- Temperature maintained at 20°C until opening of the apparatus

### 2. Results:

### 2.1. Specificity of the primers and probes when used in multiplex:

The selected IS6110 and RD9 primers and probes are MtbC-specific: they do not show a significant homology with a nucleic acid other than from a MtbC mycobacterium.
Advantageously, the selected IS6110 and RD9 primers and probe remain specific even when used in triplex.

Their "triplex-grade" specificity can be illustrated on bacterial lysates.
Presence of DNA was checked by effective amplification of 16S DNA.
A positive IC response validates the tuberculosis PCR assay.
Triplex results were as follows:

**Table 4:**

| Bacterial strains | IS6110 | RD9 | IC |
|---|---|---|---|
| 54 bacterial strains other than Mycobacterium | - | - | + |
| 26 Mycobacterium strains which do belong to the MtbC | - | - | + |
| 5 strains of M. bovis, including 4 M. bovis BCG | + | - | + |
| 23 strains of M. tuberculosis | + | + | + |
| 6 strains other than M. tuberculosis or M. bovis, but belonging to the MtbC | + | - | + |
| *(M pinnipedii, M caprae, 3 strains of M. africanum, M microti)* | | | |
| M. canetti | + | + | + |

Detailed results are shown in table 5 below.

**Table 5:**

| **Bacterial strains** | **IS6110** | **RD9** |
|---|---|---|
| *Escherichia coli,* clinical strain | - | - |
| *Citrobacter freundii,* clinical strain | - | - |
| *Salmonella typhi,* clinical strain | - | - |
| *Enterococcus faecalis,* clinical strain | - | - |
| *Enterococcus faecium,* clinical strain | - | - |
| *Corynebacterium pseudodiphtheriticum,* CIP 103420 T | - | - |
| *Corynebacterium diphteriae,* CIP 100161T | - | - |
| *Aeromonas hydrophila,* CIP 103561 | - | - |
| *Enterobacter cloacae,* CIP 103624 | - | - |
| *Staphylococcus aureus,* CIP 76.25 | - | - |
| *Pseudomonas aeruginosa,* CIP 76.10 | - | - |
| *Neisseria meningitidis,* clinical strain | - | - |
| *Serratia marcescens,* clinical strain | - | - |
| *Acinetobacter Iwoffi,* clinical strain | - | - |
| *Klebsiella pneumoniae,* CIP 104298 | - | - |
| *Enterobacter aerogenes,* CIP 103656 | - | - |
| *Streptococcus oralis,* CIP 103216 | - | - |
| *Streptococcus mutans,* CIP 103694 | - | - |
| *Streptococcus pyogenes,* CIP 104226 | - | - |
| *Streptococcus pneumoniae,* CIP 104340 | - | - |
| *Acinetobacter baumanii,* CIP 103572 | - | - |
| *Burkholderia cepacia,* CIP 80.24T | - | - |
| *Klebsiella pneumoniae subsp ozaenae,* CIP 52.212 | - | - |
| *Staphylococcus epidermidis,* ATCC14990 | - | - |
| *Haemophilus parainfluenzae,* CIP 102513T | - | - |
| *Haemophilus influenzae,* clinical strain | - | - |
| *Mycobacterium avium,* CIP 104244 | - | - |
| *Pasteurella multocida,* veterinary strain | - | - |
| *Mycoplasma pneumoniae,* clinical strain | - | - |
| *Mycobacterium aurum,* clinical strain | - | - |
| *Mycobacterium flavescens,* clinical strain | - | - |
| *Mycobacterium nonchromogenicum,* clinical strain | - | - |
| *Mycobacterium smegmatis,* clinical strain | - | - |
| *Mycobacterium paraforfuitum,* clinical strain | - | - |
| *Mycobacterium gordonae,* ATCC 14470 | - | - |
| *Mycobacterium forfuitum,* ATCC 68410 | - | - |
| *Mycobacterium kansasii,* CIP 104589 | - | - |
| *Mycobacterium phlei,* clinical strain | - | - |
| *Mycobacterium xenopi,* clinical strain | - | - |
| *Mycobacterium tuberculosis,* clinical strain | + | + |
| *Mycobacterium tuberculosis,* clinical strain | + | + |
| *Mycobacterium tuberculosis,* clinical strain | + | + |
| *Mycobacterium tuberculosis,* clinical strain | + | + |
| *Mycobacterium tuberculosis,* clinical strain | + | + |
| *Mycobacterium tuberculosis,* clinical strain | + | + |
| *Mycobacterium tuberculosis,* clinical strain | + | + |
| *Mycobacterium tuberculosis,* clinical strain | + | + |
| *Mycobacterium tuberculosis,* clinical strain | + | + |
| *Mycobacterium tuberculosis,* clinical strain | + | + |
| *Mycobacterium tuberculosis,* clinical strain | + | + |
| *Mycobacterium tuberculosis,* clinical strain | + | + |
| *Mycobacterium tuberculosis,* clinical strain | + | + |
| *Mycobacterium tuberculosis,* clinical strain | + | + |
| *Mycobacterium tuberculosis,* clinical strain | + | + |
| *Mycobacterium tuberculosis,* clinical strain | + | + |
| *Mycobacterium tuberculosis,* clinical strain | + | + |
| *Mycobacterium tuberculosis,* clinical strain | + | + |
| *Mycobacterium mac,* clinical strain | - | - |
| *Mycobacterium canetti,* clinical strain | + | + |
| *Mycobacterium bovis,* clinical strain | + | - |
| *Mycobacterium simiae,* clinical strain | - | - |
| *Mycobacterium tuberculosis,* clinical strain | + | + |
| *Mycobacterium africanum,* clinical strain | + | - |
| *Mycobacterium marinum,* clinical strain | - | - |
| *Mycobacterium tuberculosis,* CIP 64.31T | + | + |
| *Mycobacterium kansasii,* clinical strain | - | - |
| *Mycobacterium tuberculosis,* clinical strain | + | + |
| *Mycobacterium bovis BCG,* clinical strain | + | - |
| *Mycobacterium bovis* BCG, clinical strain | + | - |
| *Mycobacterium avium,* ATCC25291 | - | - |
| *Mycobacterium tuberculosis* strain H37rv, ATCC25618 | + | + |
| *Neisseria mucosa,* clinical strain | - | - |
| *Bacteroides fragilis,* CIP 77.16 | - | - |
| *Fusobacterium nucleatum,* C I P 101130 | - | - |
| *Nocardia asteroids,* clinical strain | - | - |
| *Rhodococcus equi,* clinical strain | - | - |
| *Bordetella pertussis,* clinical strain | - | - |
| *Mycoplasma orale,* ATCC23714 | - | - |
| *Mycobacterium mucogenicum,* clinical strain | - | - |
| *Mycobacterium bovis* BCG, clinical strain | + | - |
| *Mycobacterium bovis* BCG, clinical strain | + | - |
| *Mycobacterium africanum,* clinical strain | + | - |
| *Mycobacterium africanum,* clinical strain | + | - |
| *Mycobacterium africanum,* clinical strain | + | |
| *Bacteroides fragilis,* clinical strain | - | - |
| *Bacteroides fragilis,* clinical strain | - | - |
| *Bacteroides fragilis,* clinical strain | - | - |
| *Mycobacterium chelonae,* DSMZ 43804 | - | - |
| *Mycobacterium haemophilum,* DSMZ 44634 | - | - |
| *Neisseria cinerea,* clinical strain | - | - |
| *Mycobacterium celatum,* DSMZ 44243 | - | - |
| *Mycobacterium scrofulaceum,* DSMZ 43992 | - | - |
| *Mycobacterium malmoense,* DSMZ 44163 | - | - |
| *Propionibacterium acnes,* DSMZ 1897 | - | - |
| *Moraxella catarrhalis,* DSMZ 9143 | - | - |
| *Mycobacterium peregrinum,* DSMZ 43271 | - | - |
| *Eikenella corrodens,* DSMZ 8340 | - | - |
| *Micromonas micros,* DSMZ 20468 | - | - |
| *Prevotella melaninogenica,* DSMZ 7089 | - | - |
| *Veillonella parvula,* DSMZ 2007 | - | - |
| *Mycobacterium tuberculosis,* ATCC 25177 | + | + |
| *Mycobacterium microti,* ATCC 11152 | + | - |
| *Actinomyces naeslundii* ATCC 12104 | - | - |
| *Legionella pneumophila,* ATCC 33152 | - | - |
| *Streptomyces coelicolorATCC* BAA-471 | - | - |
| *Chlamydia pneumoniae,* strain CWL-029, ATCC VR- 1310 | - | - |
| *Chlamydia trachomatis,* serovar D strain UW-3/CX | - | - |
| *Lactobacillus acidophilus,* clinical strain | - | - |
| *Neisseria gonorrhoeae,* clinical strain | - | - |
| *Salmonella enteritidis,* clinical strain | - | - |
| *Legionella micdadei,* clinical strain | - | - |
| *Mycobacterium genavense,* DSMZ 44424 | - | - |
| *Mycobacterium interjectum,* DSMZ 44064 | - | - |
| *Mycobacterium saskatcewanense,* DSMZ 44616 | - | - |
| *Mycobacterium pinnipedii,* Institut Pasteur | + | - |
| *Mycobacterium caprae,* Institut Pasteur | + | - |

### 2.2. PCR sensitivity:

Triplex PCR was performed in duplicate on a lysate of pre-counted M. tuberculosis H37Rv. Results were as follows (table 6):

**Table 6:**

| | **Bacterial solution** | | **Ct** | | | | **RFU** | |
|---|---|---|---|---|---|---|---|---|
| | **CFU/ml** | **CFU/10**µ**l** | **1** | **2** | **Mean** | **Standard Deviation** | **1** | **2** |
| **IS 6110 Tamra** | 1.2 10⁷ | 1.2 10⁵ | 17.3 | 17.3 | 17.3 | 0 | 900 | 900 |
| | 1.2 10⁵ | 1.2 10³ | 23.3 | 23.4 | 23.35 | 0.07 | 875 | 875 |
| | 1.2 10³ | 12 | 31.2 | 30.3 | 30.75 | 0.63 | 500 | 575 |
| | 1.2 10² | 1.2 | 35.5 | 35.8 | 35.65 | 0.21 | 300 | 400 |
| | **12** | **0.12** | **41.3** | **41.3** | **41.3** | **0** | **150** | **200** |
| **RD9 FAM** | 1.2 10⁷ | 1.2 10⁵ | 20.3 | 19.7 | 20 | 0.42 | 2250 | 2250 |
| | 1.2 10⁵ | 1.2 10³ | 27.2 | 27 | 27.1 | 0.14 | 2000 | 1750 |
| | 1.2 10³ | 12 | 34.8 | 34.3 | 34.55 | 0.35 | 1300 | 1300 |
| | **1.2 10²** | **1.2** | **38.3** | **38.4** | **38.35** | **0.07** | **1000** | **1000** |
| | 12 | 0.12 | // | // | // | // | // | // |
| **IC ATTO 590** | 1.2 10⁷ | 1.2 10⁵ | 33.2 | 33.3 | 33.25 | 0.07 | 80 | 800 |
| | 1.2 10⁵ | 1.2 10³ | 32.2 | 31.9 | 32.05 | 0.212 | 800 | 900 |
| | 1.2 10³ | 12 | 31.3 | 31.4 | 31.35 | 0.07 | 1200 | 1200 |
| | 1.2 10² | 1.2 | 31.0 | 31.1 | 31.05 | 0.07 | 1300 | 1300 |
| | 12 | 0.12 | 31.6 | 31.1 | 31.35 | 0.35 | 1600 | 1400 |

### PCR detection threshold :

- 12 CFU/ml for IS6110, which corresponds to 0.12 copy per PCR
- 120 CFU/ml for RD9, which corresponds to 1.2 copy per PCR

The selected IS61110 and RD9 primers advantageously have a M. tuberculosis (+ the very rare M. canetti) specificity, and retain this specificity when used in multiplex. They further allow for a highly sensitive detection (almost as low as 1 copy per sample).

### Example 2 (samples collected from human beings - comparative example):

Multiplex PCR was performed as described in the above example 1, but on samples collected from human patients (Percy Hospital, France):
- sample of cerebro-spinal fluid (CSF),
- sample of bronchial lavage,
- sample of bronchial aspirate,
- sample of sputum.

Before analysis, respiratory specimens are submitted to digestion-decontamination pre-treatment.
Indeed, most specimens submitted for mycobacterial culture are contaminated with a variety of organisms that can rapidly outgrow the mycobacteria. Thus, mycobacteria are recovered optimally from clinical specimens through use of procedures that reduce or eliminate contaminating bacteria while releasing mycobacteria trapped in mucin and cells. The most widely used digestion-decontamination method is the N-Acetyl-L-Cysteine (NALC)-NaOH method. Specimens were decontaminated with 1% (final concentration) sodium hydroxide-N-acetylcysteine and concentrated by centrifugation at 3,000 × *g* for 20 min in accordance with standard procedures (Metchock, B. G., F. S. Nolte, and R. J. Wallace, Jr. 1999. Mycobacterium, p. 399-437. In P. R. Murray, E. J. Baron, M. A. Pfaller, F. C. Tenover, and R. H. Yolken (ed.), Manual of clinical microbiology, 7th ed. American Society for Microbiology, Washington, D.C.). The pellets are resuspended with phosphate buffer (0.067 M, pH 6.8). The specimens are then ready to be analyzed by microscopy test, culture or molecular biology.
The sample of cerebro-spinal fluid (CSF) are analyzed directly.

Comparative analysis was performed by:
- by bacterial cultures and microscopy, or
- with the Gen-Probe MTD test.

Bacterial cultures were performed as described in Kent and Kubica ("Public health mycobacteriology: a guide for the level III laboratory", 1985, U.S. Dpt. Of Public Health and Human Services, Atlanta, GA, USA), and in Roberts, Koneman and Kim ("Mycobacterium", 1991, pages 304-339; In A. Barlows et al. (ed.), Manual of Clinical Microbiology, American Society for Microbiology, Washington, D.C., USA).
More particularly, Lowenstein-Jensen (LJ) cultures were performed as described in Petran et al 1971. Health Lab. Sci. 8 : 225-230. LJ culture medium comprises mineral salts, potato starch, glycerine, malachite green (antiseptic), and egg (albumin). Cultures are performed in screw capped tubes. Three drops of centrifugation pellet are re-suspended in phosphate buffer and deposited on the top of the gelose slope. Two to six tubes (depending on the sample volume) are inoculated and incubated at 37°C. The tubes are loosely capped (to allow for the evaporation of any liquid in excess) and incubated in an inclined position.
Samples are observed once a week for at least 8 weeks. The observation made on week 1 allows to detect a contamination by commensal bacteria or the presence of a fast-growing mycobacterium. When a colony appears, a Ziehl-Neelsen stained smear is made to check that acid-alcohol resistant bacilli are present, before declaring that the culture is positive for mycobacterium.

Microscopy test were performed by fuchsine staining as described in Kent and Kubica ("Public health mycobacteriology: a guide for the level III laboratory", 1985, U.S. Dpt. Of Public Health and Human Services, Atlanta, GA, USA). The recommended interpretations to report smear results are given in Table 7.

**Table 7 (microscopy scores):**

| Microscopy observation (magnification x1000) | Microscopy score |
|---|---|
| direct observation is negative | Z0 |
| 1-9 bacilli per 100 fields | Z1 |
| 1-9 bacilli per 10 fields | Z2 |
| 1-9 bacilli per field | Z3 |
| more than 10 bacilli per field | Z4 |

The Gen-Probe MTD test was performed in accordance with the manufacturer's recommendations. It is implemented with the Gen-Probe® kit commercialized as "Amplified^{™} Mycobacterium tuberculosis direct test" kit (Gen-Probe Inc., San Diego, California 92121, USA). The Gen-Probe MTD test utilizes the amplification and detection of MtbC-specific rRNA. Briefly, biological samples were sonicated, subjected to heat denaturation, then submitted to amplification in accordance with the Gen-Probe TMA method (Transcription-Mediated Amplification), and submitted to amplicon detection in accordance with the Gen-Probe HPA method (Hybridization Protection Assay).
A positive MTD test indicates that the sample contain mycobacteria of the MtbC (due to problems of inhibition, and of insufficient sensitivity, a negative MTD test does however not exclude the possibility of isolating a MtbC organism from the sample).

Results were as follows:

**Table 8:**

| | **Micro scopy** | **Culture test** | | | **Gen-Probe test** | **Present invention** | | | | |
|---|---|---|---|---|---|---|---|---|---|---|
| **Sample** | **score** | **Culture result** | **Culture duration** | **Identified species** | **MtbC detection** | **RD9 (Fam)** | **IS6110 (Tamra)** | **IC (ATTO 590)** | **MtbC result** | **MtbC species** |
| | | | **(days)** | | | **Ct** | **Ct** | **Ct** | | |
| ba | Z0 | positive | **24** | *M. gordonae* | np | ND | ND | 34,7 | negative | negative |
| pus | Z3 | positive | **12** | *M. tuberculosis* | positive | 29.6 +/-0.14 | 28.5 +/-0.56 | 32,4 | positive | *M. tuberculosis** |
| ba | Z4 | positive | **13** | M. tuberculosis | positive | 35.425 +/-0.17 | 29.665 +/-0.148 | 36.235 +/-0.219 | positive | *M*. *tuberculosis** |
| bw | Z4 | positive | **13** | *M. tuberculosis* | positive | 33.1 +/-1.13 | 37.75 +/-1.34 | 32,25 | positive | *M. tuberculosis** |
| ba | Z0 | positive | **22** | *M.tuberculosis* | positive | 1 value 40.72 | 1 value 40.08 | 36.34 +/-0.18 | weak positive | *M. tuberculosis** |
| ba | Z4 | positive | **14** | *M.tuberculosis* | positive | 34.16 +/-0.09 | 28.49 +/-0.41 | 35.485 +/-0.19 | positive | *M. tuberculosis** |
| bw | Z3 | positive | **14** | *M. tuberculosis* | np | 35,1 | 32,6 +/-0,98 | 32,45 +/-0,4 | positive | M. *tuberculosis** |
| CSF | Z0 | negative | // | // | negative | ND | ND | 33,15 +/-0,21 | negative | negative |
| ba (spiked with M. africanum) | np | | | *M. africanum* | np | ND | 33,45 +/-0,77 | 31,44 +/-0,43 | positive | *MtbC other than Mt or Mc* |
| ba (spiked with M. bovis) | np | | | *M*. *bovis* | np | ND | 46,289 +/-0,44 | 30,89 +/-0,5 | positive | *MtbC other than Mt or Mc* |

| | | | | | | | | | | |
|---|---|---|---|---|---|---|---|---|---|---|
| * M. tuberculosis, or the very rare M. canetti | | | | | | | | | | |

**ND** = non detected
**np** = not performed
**MtbC other than Mt or Mc** = mycobacterium strain which belongs to the MtbC, but which is not M. tuberculosis (Mt) nor M. canetti (Mc)
**ba** = bronchial aspirate
**bw** = bronchial washing
**spiked** = sample which has been artificially infected
**CSF** = cerebro-spinal fluid

The results obtained with the primers and probes of the present invention implemented in multiplex PCR correlates with those obtained with the traditional culture method, and with the Gen-Probe kit.
The present invention is much quicker than the traditional culture method.
The present invention is also more precise than the Gen-Probe MTD test: the MTD test only gives a MtbC positive / MtbC negative response; the M. tuberculosis conclusion which is usually derived from the MTD test is only an assumption based on the fact that there is a high probability that a patient who has tuberculosis-like symptoms, and who is MTD positive, is infected by a M. tuberculosis strain. The present invention directly ascertains that the strain is M. tuberculosis (or the very rare -and easily identifiable- M. canetti), and that it is not another strain of the MtbC, and particularly that it not M. bovis (i.e., a strain which can be present in the sample due to BCG vaccination).
The present invention further allows for discrimination between those M. tuberculosis of the ancestral type (RD9+ IS6110- response), and those M. tuberculosis of the modern type (RD9+ IS6110+ response).

The content of every cited document or publication (whether patent document or scientific publication) is herein incorporated by reference.

## Claims

1. Set of two pairs of primers suitable for the specific and sensitive detection of a mycobacterium of the Mycobacterium tuberculosis complex (MtbC), and/or for the specific and sensitive discrimination of Mycobacterium tuberculosis and Mycobacterium canetti on one hand, from the other strains of said MtbC on the other hand,
wherein said two pairs of primers have such sequences that they can be used in multiplex in a single amplification run while still offering a specific and sensitive detection and discrimination,
wherein said two pairs of primers are an IS6110 primer pair consisting of an IS6110 forward primer and of an IS6110 reverse primer, and a RD9 primer pair consisting of a RD9 forward primer and of a RD9 reverse primer,
wherein said RD9 forward primer is a 5'-terminal fragment of 10 to 18 nucleotides of the sequence of SEQ ID NO:8, said 5'-terminal fragment starting from the very first 5' nucleotide of said SEQ ID NO:8,
wherein said RD9 reverse primer is a 5'-terminal fragment of 10 to 18 nucleotides of the sequence which is fully complementary of said SEQ ID NO:8 over the entire length of said SEQ ID NO:8, said 5'-terminal fragment starting from the very first 5' nucleotide of said complementary sequence,
wherein said IS6110 forward primer is a 5'-terminal fragment of 10 to 18 nucleotides of the sequence of SEQ ID NO:2, said 5'-terminal fragment starting from the very first 5' nucleotide of said SEQ ID NO:2,
wherein said IS6110 reverse primer is a 5'-terminal fragment of 10 to 18 nucleotides of the sequence which is fully complementary of said SEQ ID NO:2 over the entire length of said SEQ ID NO:2, said 5'-terminal fragment starting from the very first 5' nucleotide of said complementary sequence,
wherein said RD9 forward primer optionally comprises the sequence of SEQ ID NO:11, and/or said RD9 reverse primer optionally comprises the sequence of SEQ ID NO:12, and/or said IS6110 forward primer optionally comprises the sequence of SEQ ID NO:5, and/or said IS6110 reverse primer optionally comprises the sequence of SEQ ID NO:6.

2. Primer pair selected from the set of two primer pairs of claim 1.

3. Primer selected from a primer pair of claim 2.

4. Set of two polynucleotides suitable for use as reference template sequences in the design of primers that can be used in multiplex in a single amplification run to detect a mycobacterium of the Mycobacterium tuberculosis complex (MtbC), and/or to discriminate Mycobacterium tuberculosis and Mycobacterium canetti on one hand, from the other strains of said MtbC on the other hand,
wherein one of said two reference template polynucleotides is a fragment of RD9, the other being a fragment of IS6110,
wherein said RD9 fragment consists in the sequence of SEQ ID NO:8, or the sequence which is fully complementary to SEQ ID NO:8 over the entire length of SEQ ID NO:8,
and
wherein said IS6110 fragment consists in the sequence of SEQ ID NO:2, or in the sequence which is fully complementary to SEQ ID NO:2 over the entire length of SEQ ID NO:2.

5. Polynucleotide suitable for use as a reference template sequence in the design of primers that can be used in multiplex in a single amplification run to detect a mycobacterium of the Mycobacterium tuberculosis complex (MtbC), and/or to discriminate Mycobacterium tuberculosis and Mycobacterium canetti on one hand, from the other strains of said MtbC on the other hand,
wherein said reference template polynucleotide is selected from the set of claim 4.

6. IS6110 amplicon obtainable by using a set of two primer pairs of claim 1 as amplification primers on a biological sample, **characterized in that**:
a. it has a size ranging from 199 to 203 nucleotides, and
b. it shares a minimum of 98% sequence identity with the reference sequence of SEQ ID NO:2 or with the sequence which is fully complementary to SEQ ID NO:2 over the entire length of SEQ ID NO:2.

7. RD9 amplicon obtainable by using a set of two primer pairs of claim 1 as amplification primers on a biological sample, **characterized in that**:
a. it has a size ranging from 170 to 174 nucleotides, and
b. it shares a minimum of 98% sequence identity with the reference sequence of SEQ ID NO:8 or with the sequence which is fully complementary to SEQ ID NO:8 over the entire length of SEQ ID NO:8.

8. Probe suitable for the detection of the amplicons obtainable by using the set of two pairs of primers according to claim 1 as primers in an amplification reaction performed on a biological sample, wherein said probe is:
- the sequence of SEQ ID NO:8, or the sequence which is fully complementary to SEQ ID NO:8 over the entire length of SEQ ID NO:8, or a fragment of at least 15 nucleotides and of less than 29 nucleotides of said sequence of SEQ ID NO:8 or of said complementary sequence, or
- the sequence of SEQ ID NO:2, or the sequence which is fully complementary to SEQ ID NO:2 over the entire length of SEQ ID NO:2, or a fragment of at least 15 nucleotides and of less than 29 nucleotides of said sequence of SEQ ID NO:2 or of said complementary sequence,
said probe optionally further comprising at least one beacon arm, and/or a fluorophore at its 5'end and/or a quencher at its 3' end.

9. PCR set, consisting of at least one probe of claim 8, and of at least one primer pair of claim 2.

10. Oligonucleotide or polynucleotide suitable for use as an Internal Control (IC) in the specific and sensitive detection of a mycobacterium of the Mycobacterium tuberculosis complex (MtbC), and/or for the specific and sensitive discrimination of Mycobacterium tuberculosis and Mycobacterium canetti on one hand, from the other strains of said MtbC on the other hand, **characterized in that** said IC oligonucleotide or polynucleotide comprises:
a. two primer binding site sequences, wherein the one of said two primer binding site sequences which is located in 5' relative to the other of said two primer binding site sequences is identical to one member of a primer pair of claim 2, and the other of said two primer binding site sequences is fully complementary to the other member of the same primer pair of claim 2, and
b. a sequence which is unrelated to Mycobacterium, which is located between said two primer binding site sequences.

11. Oligonucleotide suitable for use as IC according to claim 10, **characterized in that** it has the sequence of SEQ ID NO:13.

12. Probe especially adapted to the detection of the IC oligonucleotide of claim 1**1**, **characterized in that** it has the sequence of SEQ ID NO:14.

13. Kit for the *in vitro* diagnosis of tuberculosis and/or for the specific and sensitive detection of a mycobacterium of the Mycobacterium tuberculosis complex (MtbC), and/or for the specific and sensitive discrimination of Mycobacterium tuberculosis and Mycobacterium canetti on one hand, from the other strains of said MtbC on the other hand, **characterized in that** it comprises:
- at least one set of two pairs of primers of claim 1,
- at least one primer pair of claim 2,
- at least one primer of claim 3,
- at least one set of two reference template polynucleotides of claim 4,
- at least one reference template polynucleotide of claim 5,
- at least one probe of claim 8,
said kit optionally further comprising an IC oligonucleotide or polynucleotide of claim 10 or 11, and/or the IC probe of claim 12, and/or a lysis buffer for nucleic acid extraction.

14. Method for detecting a mycobacterium of the Mycobacterium tuberculosis complex (MtbC) in a biological sample, and/or for discriminating Mycobacterium tuberculosis and Mycobacterium canetti on one hand, from the other strains of said MtbC on the other hand, **characterized in that** it comprises the detection of two target nucleic acid sequences, more particularly by PCR using the two pairs of primers of claim 1, as amplification primers, wherein one of said two target sequences is IS6110 or a fragment thereof, and the other target sequence is RD9 or a fragment thereof,
wherein the detection of the presence of said RD9 target and of the presence or absence of said IS6110 target is indicative of the presence in said biological sample of a mycobacterium belonging to the MtbC, which is either M. tuberculosis or M. canetti,
wherein the detection of the absence of said RD9 target and of the presence of said IS6110 target is indicative of the presence in said biological sample of a mycobacterium belonging to the MtbC, which is not M. tuberculosis,
wherein the detection of the absence of said RD9 target and the absence of said IS6110 target is indicative of the absence in said biological sample of a mycobacterium belonging to the MtbC,
said RD9 target sequence optionally consisting of the sequence of SEQ ID NO:8, or of the sequence which is fully complementary to SEQ ID NO:8 over the entire length of SEQ ID NO:8, and
said IS6110 target sequence optionally consisting of the sequence of SEQ ID NO:2, or of the sequence which is fully complementary to SEQ ID NO:2 over the entire length of SEQ ID NO:2.

15. *In vitro* method for the diagnosis of tuberculosis, **characterized in that** the presence or absence of a mycobacterium of the MtbC in a biological sample, and/or the presence or absence of a M. tuberculosis or M. canetti strain in a biological sample, and/or the presence or absence of a mycobacterium of the MtbC, which is not M. tuberculosis, in a biological sample, is(are) determined by implementation of the method of claim 14 on said biological sample.
